# EUROPEAN PATENT SPECIFICATION

(11) **EP 3 467 103 B1**
(45) Date of publication and mention of the grant of the patent: **04.09.2024**
(21) Application number: 17802926.0
(22) Date of filing: 26.05.2017
(51) Int. Cl.: C12N 5/0797

(54) **METHOD FOR PRODUCING NEURAL STEM CELLS, MEDIUM, SUPPLEMENT, SUPPLEMENT SET, MEDIUM KIT, AND CELL CULTURE DEVICE**
VERFAHREN ZUR HERSTELLUNG VON NEURALEN STAMMZELLEN, MEDIUM, SUPPLEMENT, SUPPLEMENTSATZ, MEDIUM-KIT UND ZELLKULTURVORRICHTUNG
PROCÉDÉ DE PRODUCTION DE CELLULES SOUCHES NEURALES, MILIEU, COMPLÉMENT, JEU DE COMPLÉMENTS, KIT DE MILIEU, ET DISPOSITIF DE CULTURE DE CELLULES

(30) Priority: 26.05.2016 JP 2016105022
(43) Date of publication of application: 10.04.2019
(73) Proprietor: Nikon Corporation, Tokyo 108-6290 (JP)
(72) Inventor: KII Hiroaki, Tokyo 108-6290 (JP); KIYOTA Yasujiro, Tokyo 108-6290 (JP); UOZUMI Takayuki, Tokyo 108-6290 (JP); FURUE Miho, Ibaraki-shi Osaka 567-0085 (JP); SUGA Mika, Ibaraki-shi Osaka 567-0085 (JP)
(74) Representative: Hoffmann Eitle
(86) International application number: PCT/JP2017/019757
(87) International publication number: WO 2017/204340

(56) References cited:
- EP-A1- 1 698 690
- EP-A1- 2 671 945
- EP-A1- 2 821 481
- WO-A1-2005/063968
- WO-A1-2016/016894
- WO-A2-2011/149762
- JP-A- 2012 175 962
- JP-A- 2013 532 961
- US-A1- 2016 068 806
- SUGA M ET AL: "A morphology-based assay platform for neuroepithelial-like cells differentiated from human pluripotent stem cells", INTERNATIONAL JOURNAL OF DEVELOPMENTAL BIOLOGY., vol. 62, no. 9-10, 2018, pages 613 - 621, XP055684302, ISSN: 0214-6282, DOI: 10.1387/ijdb.180161mf
- PICKFORD CLAIRE E. ET AL.: "Specific Glycosaminoglycans Modulate Neural Specification of Mouse Embryonic Stem Cells", STEM CELLS, vol. 29, 2011, pages 629 - 640, XP055441860
- LI JIAN ET AL.: "MEK/ERK signaling contributes to the maintenace of human embryonic stem cell self-renewal", DIFFERENTIATION, vol. 75, no. 4, 2007, pages 299 - 307, XP026765258
- KINEHARA MASAKI ET AL.: "Protein Kinase C Regulates Human Pluripotent Stem Cell Self- Renewal", PLOS ONE, vol. 8, no. 1, 2013, pages e54122, XP055105930

## Description

### Technical Field

The present invention relates to a method for producing neural stem cells, a medium, a supplement, a supplement set, a medium kit, and a cell culture device.

Priority is claimed on Japanese Patent Application No. 2016-105022, filed on May 26, 2016.

### Background Art

In recent years, attempts have been made to induce differentiation of pluripotent stem cells such as ES cells and iPS cells so that various types of tissues and cells are produced. For example, Patent Document 1, Patent Document 2, and Non-Patent Document 1 report that neural stem cells are obtained by carrying out suspension culture of embryonic stem cells to form floating aggregates.

In addition, a method (SDIA method) of differentiating embryonic stem cells by culturing the same on stromal cells is known (see Non-Patent Document 2).

On the other hand, in a cell culture process, it is important to grasp a state of cells which are being cultured. For the purpose of grasping a state of cultured cells and of achieving high throughput of evaluation, development of an imaging technology that targets cultured cells has been progressing.

### Citation List

### Patent Literature

[Patent Document 1] United States Patent No. 7,588,937
[Patent Document 2] PCT International Publication No. WO2005/123902

### Non-Patent Literature

[Non-Patent Document 1] Watanabe K, Kamiya D, Nishiyama A, Katayama T, Nozaki S, Kawasaki H, Watanabe Y, Mizuseki K, Sasai Y. "Directed differentiation of telencephalic precursors from embryonic stem cells," Nat Neurosci. 2005 Mar; 8(3): pp. 288 to 296. Epub 2005 Feb 6.
[Non-Patent Document 2] Kawasaki H, Mizuseki K, Nishikawa S, Kaneko S, Kuwana Y, Nakanishi S, Nishikawa St, Sasai Y. "Induction of midbrain dopaminergic neurons from ES cells by stromal cell-derived inducing activity." Nettiou. 2000 Oct; 28(1): pp. 31 to 40.
[Non-Patent Document 3] Krug AK, Kolde K, Gaspar JA, Rempel E, Balmer NV, Meganathan K, Vojnits K, Baquie M, Waldmann T, Ensenat-Waser R, Jagtap S, Evans RM, Julien S. Peterson H, Zagoura D, Kadereit S, Gerhard D, Sottriadou I, Heke M, Natarajan K, Henry M, Winkler J, Marchan R, Stoppini L, Bosgra S, Westerhout J, Verwei M, Vilo J, Kortenkamp A, Hescheler J, Hothorn L, Bremer S, van Thriel C, Krause KH, Hengstler JG, Rahnenfuehrer J, Leist M, Sachinidis A. "Human embryonic stem cell-derived test systems for developmental neurotoxicity : a transcriptomics approach." Arch Toxicol. 2013 Jan; 87(1): pp. 123 to 143.

### Summary of Invention

### Technical Problem

In the methods described in Patent Document 1 and the like, suspension culture of cells was carried out to create an embryoid body, and then induction of neural stem cells was carried out. According to such methods, although it is possible to produce neural stem cells, cells which are being cultured form aggregates, and it is difficult to observe the cells one by one.

In addition, in the method mentioned in Non-Patent Document 3, cells were cultured using "media in which Knock-Out Serum Replacement (KSR) was added to DMEM-F12". However, in these media, many ingredients are blended, and there may be cases where it is inconvenient to use these media for imaging such as a case where these ingredients absorb light for observation.

The present invention has been made in view of such circumstances, and an object of the present invention is to provide the use of a supplement, a supplement set for inducing neural stem cells, a medium kit for inducing neural stem cells, a medium, and a method for producing neural stem cells, which can be used for induction of neural stem cells and are also suitable for imaging.

### Solution to Problem

That is, the present invention includes the following aspects.

According to an aspect of the present invention, there is provided a method for producing neural stem cells, the method including a culture step of culturing a cell using a medium that contains a TGF-β receptor inhibitor and a protein kinase C inhibitor.

According to another aspect of the present invention, there is provided a method for producing neural stem cells, the method including a first culture step of culturing a cell using a first medium that does not contain a TGF-β receptor inhibitor and a protein kinase C inhibitor, and a second culture step of culturing the cell using a second medium that contains a TGF-β receptor inhibitor and a protein kinase C inhibitor, after the first culture step.

According to still another aspect of the present invention, there is provided a method for producing neural stem cells, the method including a first culture step of culturing a cell using a first medium that contains ascorbic acid or an ascorbic acid derivative, and a second culture step of culturing the cell using a second medium that contains a TGF-β receptor inhibitor and a protein kinase C inhibitor, after the first culture step.

According to still another aspect of the present invention, there is provided a method for producing neural stem cells, the method including a differentiation induction step of inducing differentiation by culturing a cell using a differentiation induction medium that contains a TGF-β receptor inhibitor and a protein kinase C inhibitor, and does not contain ascorbic acid or an ascorbic acid derivative, and a subculture step of subculturing the cell using a subculture medium that contains a TGF-β receptor inhibitor, a protein kinase C inhibitor, and ascorbic acid or an ascorbic acid derivative, after the differentiation induction step.

According to still another aspect of the present invention, there is provided a method for producing neural stem cells, the method including a first culture step of culturing a cell using a first medium that does not contain a TGF-β receptor inhibitor and a protein kinase C inhibitor, a second culture step of culturing the cell using a second medium that contains a TGF-β receptor inhibitor and a protein kinase C inhibitor, and does not contain ascorbic acid or an ascorbic acid derivative, after the first culture step, and a third culture step of culturing the cell using a third medium that contains a TGF-β receptor inhibitor, a protein kinase C inhibitor, and ascorbic acid or an ascorbic acid derivative, after the second culture step.

According to still another aspect of the present invention, there is provided a method for producing neural stem cells, the method including a first culture step of culturing a cell using a first medium that contains ascorbic acid or an ascorbic acid derivative, a second culture step of culturing the cell using a second medium that contains a TGF-β receptor inhibitor and a protein kinase C inhibitor, and does not contain ascorbic acid or an ascorbic acid derivative, after the first culture step, and a third culture step of culturing the cell using a third medium that contains a TGF-β receptor inhibitor, a protein kinase C inhibitor, and ascorbic acid or an ascorbic acid derivative, after the second culture step.

According to still another aspect of the present invention, there is provided the use of a medium which contains a TGF-β receptor inhibi or and a protein kinase C inhibitor.

According to still another aspect of the present invention, there is provided the use of a medium which includes a basal medium, a TGF-β receptor inhibitor, a protein kinase C inhibitor, insulin, selenic acid, basic fibroblast growth factor, and heparan sulfate.

According to still another aspect of the present invention, there is provided the use of a medium which includes a basal medium, a TGF-β receptor inhibitor, a protein kinase C inhibitor, insulin, selenic acid, basic fibroblast growth factor, heparan sulfate, and ascorbic acid or an ascorbic acid derivative.

According to still another aspect of the present invention, there is provided the use of a medium which contains insulin and selenic acid, and does not contain a TGF-β receptor inhibitor and a protein kinase C inhibitor.

According to still another aspect of the present invention, there is provided the use of a medium which contains insulin, selenic acid, and ascorbic acid or an ascorbic acid derivative.

According to still another aspect of the present invention, there is provided the use of a medium which includes a basal medium, insulin, selenic acid, basic fibroblast growth factor, and ascorbic acid or an ascorbic acid derivative.

According to still another aspect of the present invention, there is provided the use of a supplement which contains a TGF-β receptor inhibitor and a protein kinase C inhibitor.

According to still another aspect of the present invention, there is provided the use of a supplement which includes a TGF-β receptor inhibitor, a protein kinase C inhibitor, insulin, selenic acid, basic fibroblast growth factor, and heparan sulfate.

According to still another aspect of the present invention, there is provided the use of a supplement which includes a TGF-β receptor inhibitor, a protein kinase C inhibitor, insulin, selenic acid, basic fibroblast growth factor, heparan sulfate, and ascorbic acid or an ascorbic acid derivative.

According to still another aspect of the present invention, there is provided the use of a supplement which contains insulin and selenic acid, and does not contain a TGF-β receptor inhibitor and a protein kinase C inhibitor.

According to still another aspect of the present invention, there is provided the use of a supplement which contains insulin, selenic acid, and ascorbic acid or an ascorbic acid derivative.

According to still another aspect of the present invention, there is provided the use of a supplement which includes insulin, selenic acid, basic fibroblast growth factor, and ascorbic acid or an ascorbic acid derivative.

According to still another aspect of the present invention, there is provided the use of a supplement set which contains any of the supplements.

According to still another aspect of the present invention, there is provided the use for the induction of differentiation from a cell into a neural stem cell of a medium kit which contains a basal medium and any of the supplements as defined in the claims.

According to still another aspect of the present specification, there is provided a cell culture device which includes an incubator for culturing a pluripotent stem cell, imaging means for imaging the cell cultured in the incubator; and medium replacement means for replacing any of the media with the other medium in the incubator.

In addition, one embodiment of the present invention provides the following (1) to (9).

(1) In the embodiment of the present invention, a second supplement for inducing neural stem cells is a supplement which is used by being added to a medium for further culturing cells in a substantially monolayer state, the cells having been cultured using a medium that contains a first supplement to be added to a medium for culturing cells in a substantially monolayer state, and which is capable of inducing neural stem cells in a substantially monolayer state from the cells.
(2) in the embodiment of the present invention, a supplement set for inducing neural stem cells includes
   a first supplement to be used by being added to a medium for culturing cells in a substantially monolayer state, and
   the above-described second supplement for inducing neural stem cells.
(3) In the embodiment of the present invention, a third supplement is a supplement which is used by being added to a medium for further culturing neural stem cells which are in a substantially monolayer state, the neural stem cells having been obtained by further culturing cells, which are in a substantially monolayer state, and have been cultured using a medium that contains a first supplement to be added to a medium for culturing cells in a substantially monolayer state, in a medium containing the above-described second supplement for inducing neural stem cells.
(4) In the embodiment of the present invention, a supplement set for inducing neural stem cells is the above-described supplement set for inducing neural stem cells which further includes the above-described third supplement.
(5) In the embodiment of the present invention, a medium kit for inducing neural stem cells includes
   the above-described second supplement for inducing neural stem cells, the above-described supplement set, the above-described third supplement, or the above-described supplement set, and
   a basal medium.
(6) In the embodiment of the present invention, a second medium contains a basal medium and the above-described second supplement.
(7) In the embodiment of the present invention, a third medium is a medium which is used for further culturing neural stem cells which are in a substantially monolayer state, the neural stem cells having been obtained by further culturing cells, which are in a substantially monolayer state and have been cultured using a first medium that contains a first supplement to be added to a medium for culturing cells in a substantially monolayer state, in the above-described second medium, and which contains a basal medium and the above-described third supplement.
(8) In the embodiment of the present invention, a method for producing neural stem cells includes
   a first culture step of culturing pluripotent stem cells in a substantially monolayer state using a first medium that contains a first supplement to be added to a medium for culturing cells in a substantially monolayer state, and
   a second culture step of culturing the cells, which have been cultured in the first culture step, using the above-described second medium, to obtain neural stem cells in a substantially monolayer state.
(9) In the embodiment of the present specification, a cell culture device includes
   an incubator for culturing pluripotent stem cells,
   imaging means for imaging the cells cultured in the incubator, and
   medium replacement means for replacing the first medium that contains the first supplement to be added to a medium for culturing cells in a substantially monolayer state with the above-described second medium in the incubator.

### Brief Description of Drawings

FIG. 1 is a diagram showing steps in a method for producing neural stem cells of the embodiment.
FIG. 2 is a schematic diagram showing a procedure of a pluripotency-maintaining culture step in the method for producing neural stem cells of the embodiment.
FIG. 7 is a schematic diagram showing a procedure of the first culture step and the second culture step in the method for producing neural stem cells of the embodiment.

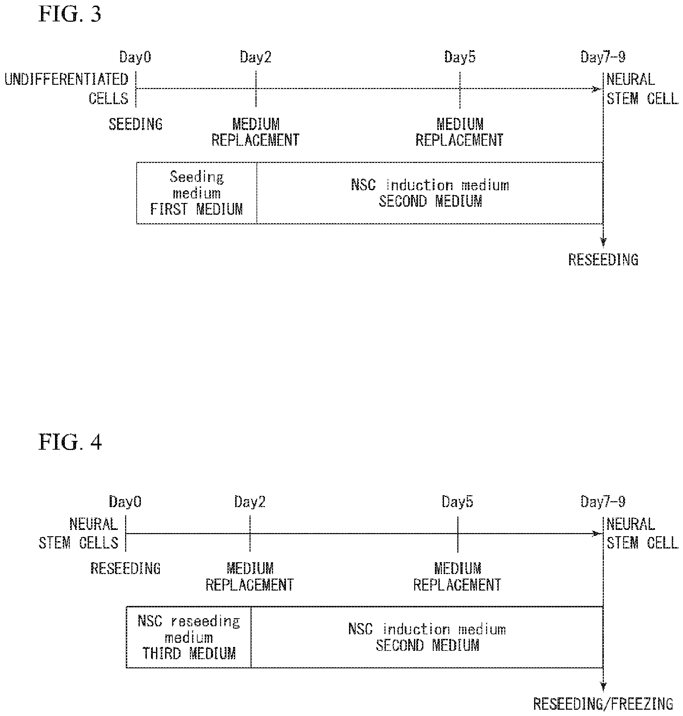

FIG. 4 is a schematic diagram showing a procedure of the third culture step and the second culture step in the method tor producing neural stem cells of the embodiment.

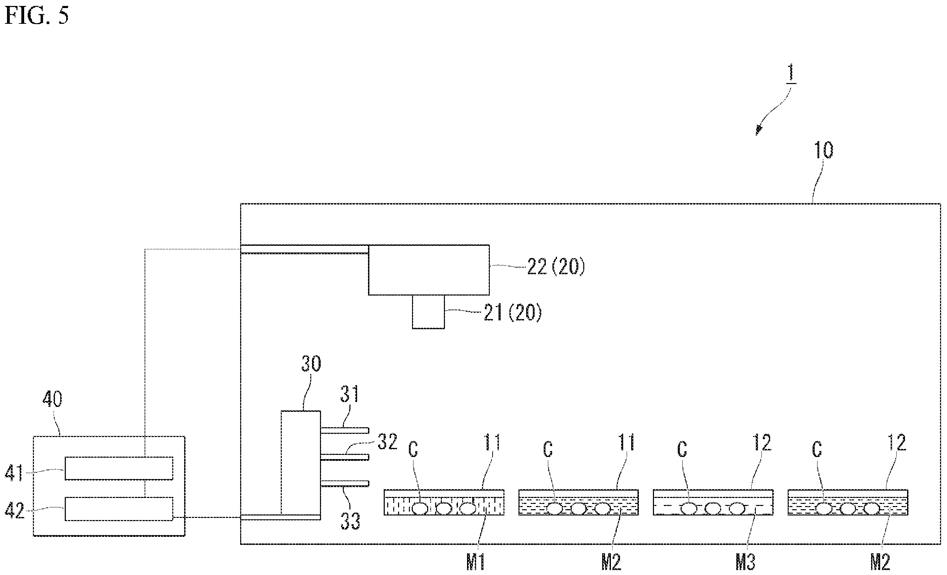

FIG. 5 is a schematic diagram showing an example of a configuration of a cell culture device of the embodiment.

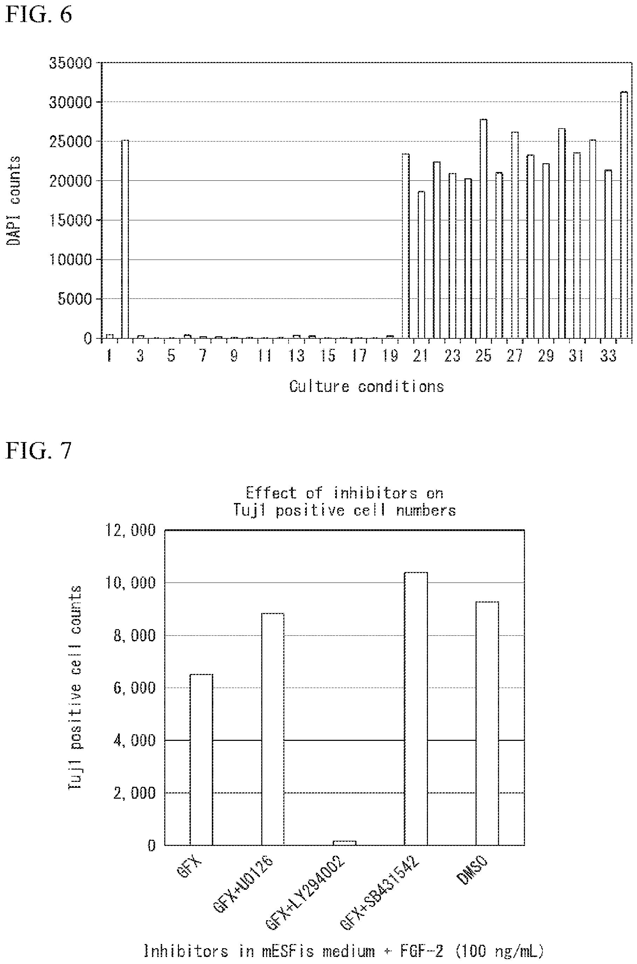

FIG. 6 is a graph showing results of examining influences of candidate ingredients of supplements on cells in Examples.
FIG. 7 is a graph showing results of examining influences of candidate ingredients of supplements on cells in Examples.

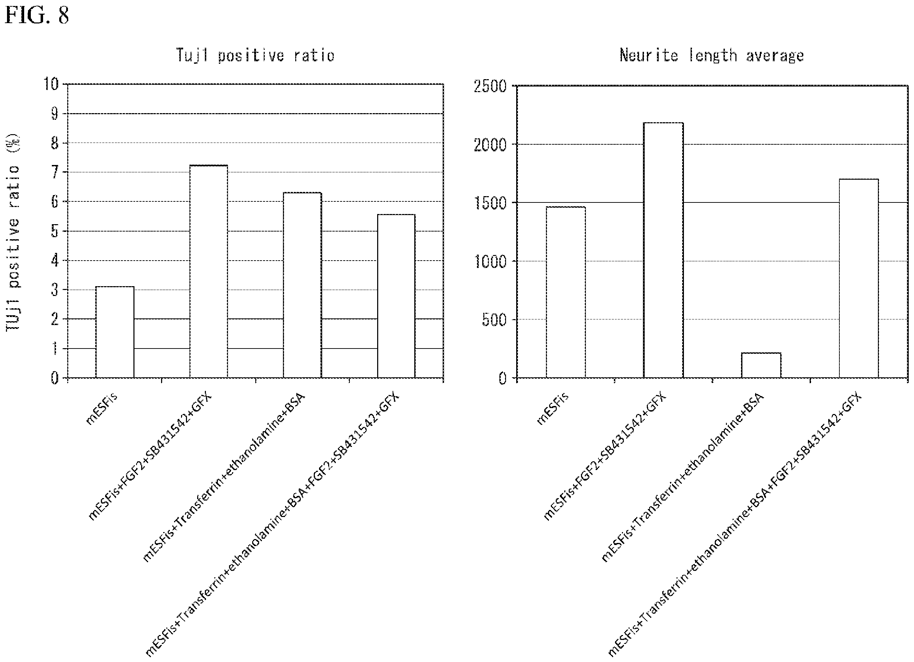

FIG. 8 is a graph showing results of examining influences of candidate ingredients of supplements on cells in Examples.

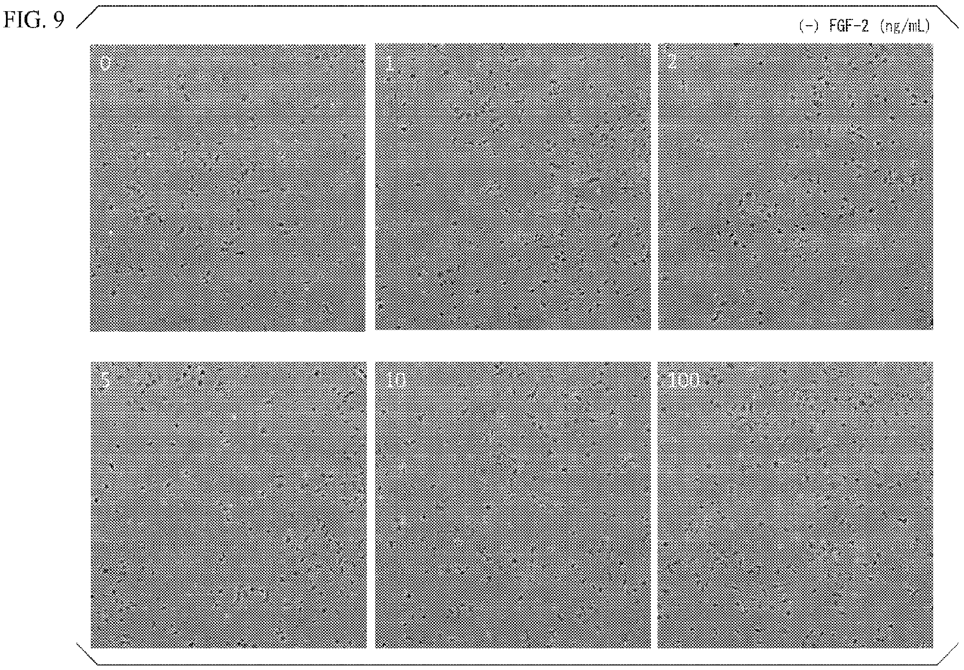

FIG. 9 is phase contrast microscope photographs showing results of examining influences of candidate ingredients of supplements on cells in Examples.

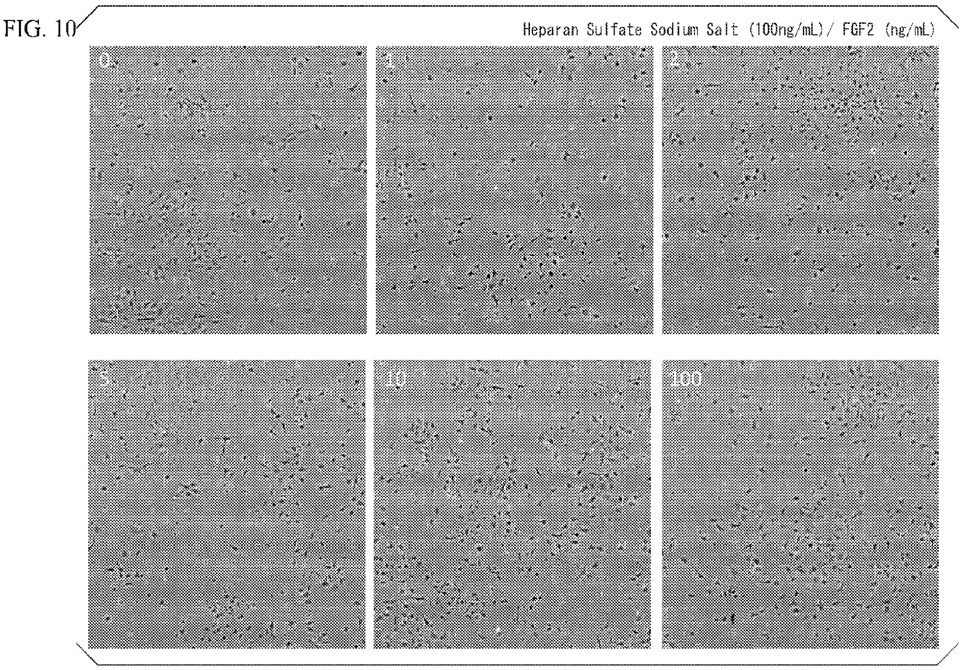

FIG. 10 is phase contrast microscope photographs showing results of examining influences of candidate ingredients of supplements on cells in Examples.

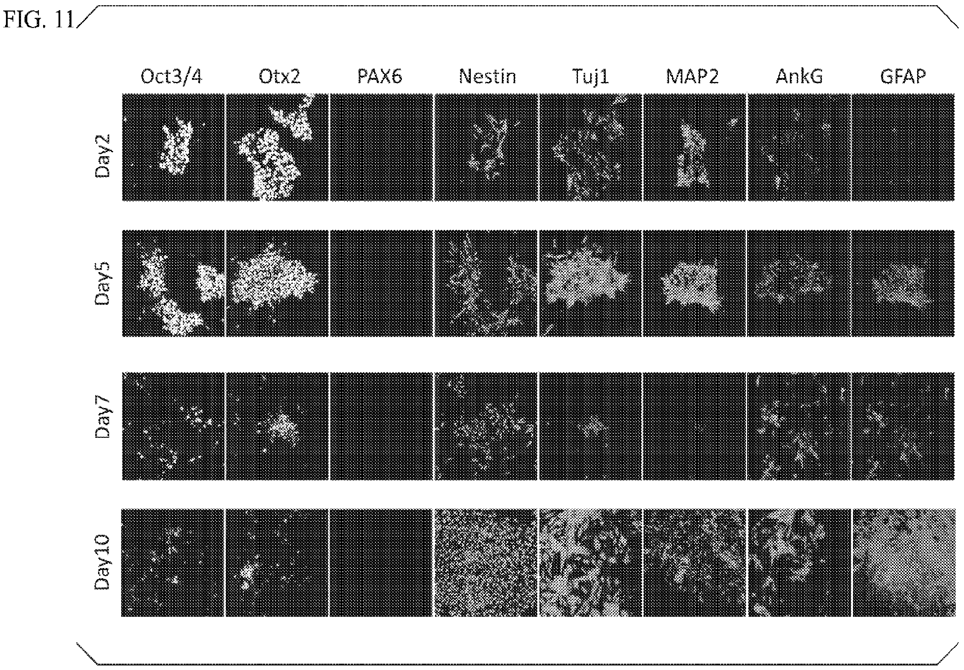

FIG. 11 is immunostaining photographs showing expression of various markers in cells which are being cultured, the photographs being acquired in Examples

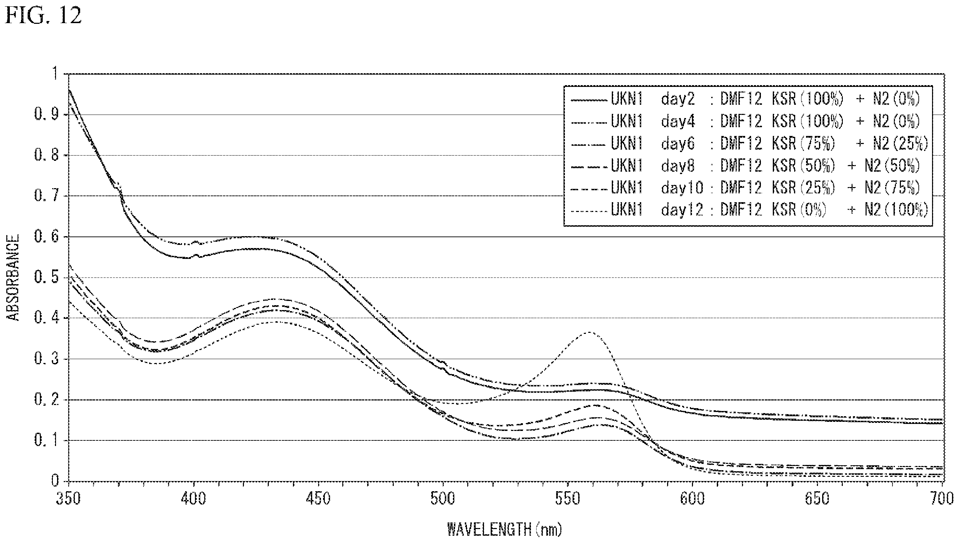

FIG. 12 is a graph showing absorbance values of media which are acquired in Examples.

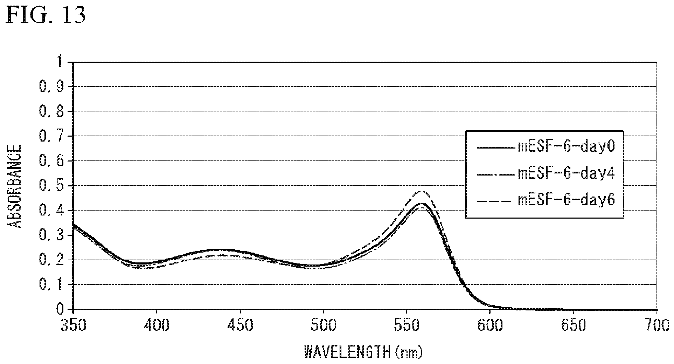

FIG. 13 is a graph showing absorbance values of media which are acquired in Examples.

### Description of Embodiments

### <<Supplement, supplement set for inducing neural stem cells, medium kit for inducing neural stem cells, medium set for inducing neural stem cells>>

Hereinafter, a supplement, a supplement set for inducing neural stem cells, a medium kit for inducing neural stem cells, a medium, and a medium set for inducing neural stem cells of the embodiment will be described. These can be used for production of neural stem cells and the like.

### <Supplement>

First, cells in a substantially monolayer state can be cultured in a medium that contains the first supplement of the embodiment to be added to a medium for culturing cells in a substantially monolayer state. By further culturing the cells using a medium that contains the second supplement of the embodiment, it is possible to induce neural stem cells in a substantially monolayer state.

The first, second, and third supplements of the embodiment preferably contain substances which are partially common to one other, in culture steps. In addition, the first, second, and third supplements of the embodiment preferably use, as a base, a supplement to be added to a medium which is used in a case of inducing neural stem cells. In addition, the supplement preferably contains a compound.

The first supplement of the embodiment may contain insulin, selenic acid, basic fibroblast growth factor, and ascorbic acid or an ascorbic acid derivative.

The second supplement of the embodiment may contain insulin, selenic acid, basic fibroblast growth factor, a TGF-β receptor inhibitor, and a protein kinase C inhibitor The second supplement may further contain heparan sulfate

The third supplement of the embodiment may contain insulin, selenic acid, basic fibroblast growth factor, a TGF-β receptor inhibitor, a protein kinase C inhibitor, and ascorbic acid or an ascorbic acid derivative. The third supplement may further contain heparan sulfate.

The second supplement can be used by being added to a medium for further culturing cells which have been cultured using a medium that contains the first supplement.

The third supplement can be used by being added to a medium for further culturing neural stem cells that have been obtained by further culturing cells, which had been cultured in a medium that contains the first supplement, in a medium containing the second supplement.

In the embodiment of the present invention, the supplement set for inducing neural stem cells includes the first supplement and the second supplement.

In the embodiment of the present invention, the supplement set for inducing neural stem cells includes the first supplement, the second supplement, and the third supplement.

### <Medium kit>

In the present embodiment, each of the first supplement, the second supplement, and the third supplement can be added to a basal medium capable of culturing cells and used for culturing the cells.

Therefore, each of the supplements and the basal medium may be provided as a kit.

In the embodiment of the present invention, a medium kit for inducing neural stem cells includes the basal medium, the first supplement, and the second supplement.

In the embodiment of the present invention, a medium kit for inducing neural stem cells includes the basal medium, the first supplement, the second supplement, and the third supplement.

### <Medium and medium set>

Each of a first medium that contains the basal medium and the first supplement, a second medium that contains the basal medium and the second supplement, a third medium that contains the basal medium and the third supplement can be used for producing neural stem cells.

Therefore, each of the supplements and the basal medium may be provided as a medium obtained by mixing each other in advance.

The first medium of the embodiment contains the basal medium and the first supplement.

The second medium of the embodiment contains the basal medium and the second supplement.

The third medium of the embodiment contains the basal medium and the third supplement.

The second medium can be used for further culturing the cells which have been cultured using the first medium.

The second medium can be used for further culturing the neural stem cells that have been obtained by further culturing the cells, which had been cultured using the first medium, in the second medium.

In the embodiment of the present invention, the medium set for inducing neural stem cells includes the first medium that contains the basal medium and the first supplement, and the second medium that contains the basal medium and the second supplement.

In the embodiment of the present invention, the medium set for inducing neural stem cells includes the first medium that contains the basal medium and the first supplement, the second medium that contains the basal medium and the second supplement, and the third medium that contains the basal medium and the third supplement.

In addition, according to the respective media of the first medium, the second medium, and the third medium of the embodiment, it is possible to culture cells in a substantially monolayer state. According to the first medium and the second medium of the embodiment, neural stem cells in a substantially monolayer state can be produced so that images of individual cells can be acquired accurately, and a state of the cells can be grasped so that the neural stem cells can be produced efficiently.

In the present specification, cells "in a monolayer state" refer to cells being in a state where the cells adhere on a bottom surface or wall surface of a culture container or instrument, and do not overlap with one another.

It is most preferable that cells are in a monolayer state. However, cells may be in a substantially monolayer state in which there is a part where the cells overlap with one another in a partial region. In this case, a maximum value of overlapping cells is preferably about 10 or less, and more preferably about 5 or less. Cells "in a substantially monolayer state" refer to cells being in a state where an average value in thickness of a cell layer in a cell assembly which is being cultured is less than 5 times, preferably less than 2 times, and more preferably less than 1.5 times an average value in thickness of one cell. The average value in thickness of one cell and the average value in thickness of the cell layer are obtained as an arithmetic mean at 10 or more places randomly extracted from the cell assembly which is being cultured.

Although there are cases where some differences may occur depending on a state of cells, that is, whether the cells are pluripotent stem cells or neural stem cells, a condition of being in a substantially monolayer state is preferable. Also, although there are cases where some differences may occur in conditions of images suitable for observation depending on an observation condition, for example, whether the condition is phase difference observation or fluorescence observation, a condition of being in a substantially monolayer state is preferable.

In the present specification, "monolayer culture" refers to culturing cells in the monolayer state or substantially monolayer state as described above.

Hereinafter, the respective ingredients according to the embodiment will be described.

### (Insulin)

Insulin may be naturally derived insulin such as porcine-derived insulin, bovine-derived insulin, and human-derived insulin. In addition, the insulin may be gene-recombinant insulin such as bovine type, porcine type, and human type, and the like, within a range usable for inducing neural stem cells in the first medium, the second medium, and the third medium, and, in particular, human type gene-recombinant insulin (recombinant human insulin) is a suitable exemplary example.

In the first medium, the second medium, and the third medium, the insulin may be contained in an amount of 1 to 20 mg/L, may be contained in an amount of 5 to 15 mg/L, may be contained in an amount of 7.5 to 12.5 mg/L, or may be contained in an amount of 9 to 11 mg/L, at a final concentration.

### (Selenic acid)

As selenic acid, selenic acid and a derivative thereof, and salts and hydrates thereof can be included, and selenic acid, sodium selenate, sodium selenite, sodium hydrogen selenite, and the like can be mentioned, with sodium selenite being preferable.

In the first medium, the second medium, and the third medium, selenic acid may be contained in an amount of 1 to 100 nmol/L, may be contained in an amount of 5 to 50 nmol/L, may be contained in an amount of 10 to 40 nmol/L, or may be contained in an amount of 10 to 25 nmol/L, at a final concentration.

### (Basic fibroblast growth factor)

Basic fibroblast growth factor (bFGF) is also called fibroblast growth factor-2 (FGF-2). The bFGF may be naturally derived FGF-2 such as porcine-derived FGF-2, bovine-derived FGF-2 and human-derived FGF-2. In addition, the bFGF may be gene-recombinant human FGF-2 such as bovine type, porcine type, and human type, and the like, within a range usable for inducing neural stem cells in the first medium, the second medium, or the third medium, and, in particular, human type gene-recombinant FGF-2 (recombinant human FGF-2 (rhFGF-2)) is a suitable exemplary example. In addition, within a range usable for inducing neural stem cells, bFGF derivatives in which a part of an amino acid sequence thereof is deleted or substituted with other amino acids, another amino acid sequence is partially inserted, one or two or more amino acids are bound to N-terminal and/or C-terminal, or a saccharide chain thereof is deleted or substituted can be included.

In the first medium, the second medium, and the third medium, the bFGF may be contained in an amount of 0.1 to 200 µg/L, may be contained in an amount of 1 to 100 µg/L, may be contained in an amount of 1 to 50 µg/L, or may be contained in an amount of 3 to 20 µg/L, at a final concentration.

In a case where heparan sulfate is contained in the second medium or the third medium, in the second medium or the third medium, the bFGF may be contained in an amount of 1 to 50 µg/L or may be contained in an amount of 3 to 20 µg/L, at a final concentration.

### (Ascorbic acid)

As ascorbic acid, in addition to ascorbic acid, within a range usable for inducing neural stem cells in the first medium or the third medium, ascorbic acid derivative such as ascorbic acid 2-phosphoric acid ester (ascorbic acid 2-phosphateester), magnesium ascorbyl phosphate, sodium ascorbyl sulfate, ascorbyl aminopropyl phosphate, and sodium ascorbyl phosphate may be included.

In the first medium or the third medium, ascorbic acid may be contained in an amount of 0.001 to 5 g/L, may be contained in an amount of 0.01 to 1 g/L, may be contained in an amount of 0.05 to 0.5 g/L, and may be contained in an amount of 0.07 to 0.3 g/L, at a final concentration.

### (TGF-β receptor inhibitor)

The TGF-β receptor inhibitor is a substance that inhibits a function of a TGF-β receptor, and may be a substance that acts on the TGF-β receptor and inhibits a function of the TGF-β receptor. The TGF-β receptor may be TGF-β type 1 receptor of the TGF-β or ALK5 of the TGF-β type 1 receptor of the TGF-β. The TGF-β receptor inhibitor may inhibit signal transduction of a Smad signaling pathway. Whether a substance inhibits the function of the TGF-β receptor can be evaluated by a known method. For example, in a case where a comparison is made between cells to which a test substance has been administered and cells to which the test substance has not been administered, and the function of the TGF-β receptor is inhibited in the cells to which the test substance has been administered, it can be determined that the test substance is a TGF-β receptor inhibitor.

As the TGF-β receptor inhibitor, a commercially available compound may be used, and, for example, trade name: SB 431542 (Cayman Chemical Company, molecular formula: C₂₂H₁₆N₄O₃, CAS No: 301836-41-9), trade name: D4476 (Wako Pure Chemical Industries, Ltd., molecular formula: C₂₃H₁₈N₄O₃, CAS No. 301836-43-1), and trade name: LY2157299 (Wako Pure Chemical Industries, Ltd., molecular formula: C₂₂H₁₉N₅O, CAS No: 700874-72-2) is an exemplary example.

In the second medium or the third medium, the TGF-β receptor inhibitor may be contained in an amount of 0.1 to 100 µmol/L, may be contained in an amount of 0.5 to 50 µmol/L, or may be contained in an amount of 1 to 20 µmol/L, or may be contained in an amount of 5 to 15 µmol/L, at a final concentration.

### (Protein kinase C inhibitor)

The protein kinase C (PKC) inhibitor is a substance that inhibits a function of PKC and may be a substance that acts on the PKC and inhibits the function of the PKC. Whether a substance inhibits the function of the PKC can be evaluated by a known method. For example, in a case where a comparison is made between cells to which a test substance has been administered and cells to which the test substance has not been administered, and the function of the PKC is inhibited in the cells to which the test substance has been administered, it can be determined that the test substance is a PKC inhibitor.

As the PKC inhibitor, a commercially available compound may be used, and, specifically, myristoylated protein kinase C peptide inhibitor (manufactured by Promega), calphostin which is staurosporine or a staurosporine derivative, 4'-N-benzoylstaurosporine, bisindolylmaleimides such as GF109203X, for example, bisindolylmaleimide 1, (isoquinolinesulfonyl)-2-methylpiperazine dihydrochloride (H-7), N-[2-(methylamino)ethyl]-5-isoquinolinesulfonamide (H-8), N-(2-aminoethyl)-5-isoquinolinesulfonamide (H-9), and the like are exemplary examples.

In the second medium or the third medium, the PKC inhibitor may be contained in an amount of 0.1 to 100 µmol/L, may be contained in an amount of 0.5 to 50 µmol/L, may be contained in an amount of 1 to 10 µmol/L, or may be contained in an amount of 1.5 to 5 µmol/L, at a final concentration

### (Heparan sulfate)

As the heparan sulfate, heparin which is glycosaminoglycan, a polymer of uronic acid and D-glucosamine, and is a factor having an anticoagulant activity can be mentioned. Regardless of being natural or synthetic products, within a range usable for inducing neural stem cells in the second medium, or the third medium, derivatives of the heparan sulfate, and salts and hydrates thereof can be included, and heparin sodium salt or heparin calcium salt which is chemically synthesized by a conventional method are exemplary suitable examples.

In the second medium or the third medium, the heparan sulfate may be contained in an amount of 10 to 50,000 µg/L, may be contained in an amount of 30 to 1,000 µg/L, may be contained in an amount of 50 to 500 µg/L, or may be contained in an amount of 80 to 200 µg/L, at a final concentration.

### (Basal medium)

The basal medium used in the embodiment is a medium which is blended in the first medium and the second medium and is capable of inducing neural stem cells from pluripotent stem cells, and is not particularly limited as long as the basal medium is blended in the third medium and is capable of culturing neural stem cells. The basal medium preferably contains one or two or more types of saccharide(s), one or two or more types of inorganic salt(s), one or two or more types of amino acid(s), one or two or more types of vitamin(s), and one or two or more types of trace ingredient(s). In addition, the basal medium can also appropriately contain antibiotics such as kanamycin for use in a drug susceptibility test.

As the saccharides, specifically monosaccharides such as glucose, lactose, mannose, fructose and galactose, and disaccharides such as sucrose, maltose, and lactose can be mentioned. Among these, glucose may be used, and one or two or more of these saccharides may be contained in the basal medium.

As the inorganic salts, specifically, combinations including calcium chloride, calcium nitrate, copper sulfate pentahydrate, iron (III) nitrate nonahydrate, iron (II) sulfate heptahydrate, magnesium chloride hexahydrate, magnesium sulfate, potassium chloride, sodium chloride, disodium hydrogen phosphate, disodium hydrogen phosphate dihydrate, sodium dihydrogen phosphate, sodium dihydrogen phosphate dihydrate, and zinc sulfate heptahydrate can be mentioned, and one or two or more of these inorganic salts may be contained in the basal medium.

As the amino acids, specifically, preferably L-amino acids of alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, valine, and the like, and derived products such as derivatives thereof, and salts and hydrates thereof can be included. For example, as the arginine, derived products of arginine such as L-arginine hydrochloride and L-arginine monohydrochloride can be included. As the aspartic acid, derived products of aspartic acid such as L-aspartic acid sodium salt monohydrate, L-aspartic acid monohydrate, L-aspartic acid potassium, and L-aspartic acid magnesium can be included. As the cysteine, derived products of cysteine such as L-cysteine dihydrochloride and L-cysteine hydrochloride monohydrate can be included. As the glutamic acid, derived products of glutamic acid such as L-glutamic acid monosodium salt can be included. As the asparagine, derived products of asparagine such as L-asparagiiie monohydrate can be included. As the tyrosine, derived products of tyrosine such as L-tyrosine disodium dehydrate may be included. As the histidine, derived products of histidine such as histidine hydrochloride and histidine hydrochloride monohydrate may be included. As the lysine, derived products of lysince such as L-lysine hydrochloride can be included. One or two or more of these amino acids may be contained in the basal medium.

As the vitamins, specifically, one or more vitamins selected from ascorbic acid, biotin, choline, folic acid, inositol, niacin, pantothenic acid, pyridoxine, riboflavin, thiamine, vitamin B12, and paraaminobenzoic acid (PABA), and derived products such as derivatives of these respective ingredients, and salts and hydrates thereof can be included. For example, as the ascorbic acid, derived products of ascorbic acid such as ascorbic acid 2-phosphoric acid ester (ascorbic acid 2-phosphate), magnesium ascorbyl phosphate, sodium ascorbyl sulfate, ascorbyl aminopropyl phosphate, and sodium ascorbyl phosphate can be included. As the choline, derived products of choline such as choline chloride can be included. As the niacin, derived products of niacin such as nicotinic acid, nicotinic acid amide, and nicotinic alcohol can be included. As the pantothenic acid, derived products of pantothenic acid such as calcium pantothenate, sodium pantothenate, and panthenol can be included. As the pyridoxine, derived products of pyridoxine such as pyridoxine hydrochloride, pyridoxal hydrochloride, pyridoxal phosphate, and pyridoxamine can be included. As the thiamine, derived products of thiamine such as thiamine hydrochloride, thiamine nitrate, bisthiamine nitrate, thiamin dicetyl sulfuric acid ester salt, fursultiamine hydrochloride, octothiamine, and benfotiamine can be included. One or two or more of these vitamins may be contained in the basal medium.

The above-mentioned ascorbic acid or ascorbic acid derivative may be contained in the basal medium. On the other hand, however, it is preferable that the first supplement and the third supplement contain the ascorbic acid or ascorbic acid derivative, and furthermore, the second supplement and the second medium do not contain the ascorbic acid or ascorbic acid derivative. From this viewpoint, it is preferable that the ascorbic acid or ascorbic acid derivative is not contained in the basal medium.

As the trace ingredient, ingredients used as usual medium ingredients such as glutathione, hypoxanthine, lipoic acid, linolenic acid, phenol red, putrescine, pyruvic acid, thymidine, and NaHCO₃, and derivatives thereof, and derived products such as salts and hydrates thereof can be included. For example, putrescine dihydrochloride, sodium pyruvate, and the like can be mentioned. One or two or more of these trace ingredients may be contained in the basal medium.

As specific examples of the basal medium, a medium such as a commercially available Dulbecco's Modified Eagle's Medium (DMEM), a Minimum Essential Medium (MEM), Eagle's Basal Medium (BME), and a known chemically-synthesized medium such as RPMT 1640 medium and F12 medium, RD medium (a medium in which the RPMI 1640 medium and the DMEM are mixed at a ratio of 1:1), and DMEM/F12 medium (a medium in which the DMEM and the F12 medium are mixed at 1:1) are mentioned. The basal medium may be a medium in which any two or more of these media are mixed at an appropriate ratio. The basal medium is preferably a medium for pluripotent stem cells. The medium for pluripotent stem cells refers to a medium which is capable of growing and culturing pluripotent stem cells while maintaining undifferentiation potency thereof by causing an additional factor for maintaining usdifferentiation to be added thereto, if necessary. As examples of preferable medium for pluripotent stem cells, in addition to the basal medium (mESF) shown in Examples as described later, hESF medium, DMEM medium, and DMEM/F12 medium are exemplary examples.

In the first supplement, the second supplement, or the third supplement, the respective ingredients of insulin, selenic acid, basic fibroblast growth factor, ascorbic acid or an ascorbic acid derivative, and heparan sulfate may be blended in an overlapping manner. However, these respective ingredients may be the same as or different from one another among the supplements.

In the first medium, the second medium, and the third medium, the basal medium is blended in an overlapping manner. However, these basal media may be the same as or different from one another among the respective media.

For the basal medium included in the medium kit for inducing neural stem cells, one type or a plurality of types may be used. As an example of a case where a plurality of types of basal media are included, causing the respective basal media for mixing to be included for the respective supplements is mentioned. For example, in a case where a medium kit for inducing neural stem cells includes a first supplement, a second supplement, and a third supplement as supplements, the basal medium may have a first basal medium to be mixed with the first supplement, a second basal medium to be mixed with the second supplement, and a third basal medium to be mixed with the third supplement.

It is preferable that the first supplement, the first medium, the second supplement, the second medium, the third supplement, and the third medium as mentioned above do not contain albumin. In the related art, a medium for inducing neural stem cells usually contains approximately 2,000 mg/L of albumin. On the other hand, the medium of the present embodiment allows good induction of neural stem cells to be achieved even without containing albumin.

In the first medium, the second medium and the third medium, the amount of albumin may be 0 to 1,000 mg/L, may be 0 to 100 mg/L, or may be 0 to 20 mg/L, or substantially no albumin may be contained.

As albumin, naturally derived albumin such as ovalbumin, porcine derived albumin, bovine derived albumin, and human derived albumin, gene-recombinant albumin such as bovine type, porcine type, and human type as albumin of animal product-free grade, and the like are mentioned.

Decreasing the amount of albumin in the medium can cause a value of absorbance of the medium to be decreased. Thus, there is little possibility that a medium ingredient affects image acquisition, observation can be made stably, and imaging is also suitably done.

It is preferable that the first supplement, the first medium, the second supplement, the second medium, the third supplement, and the third medium as mentioned above do not contain 2-mercaptoethanol (2-ME). In the related art, a medium used for culturing stem cells may contain 2-ME. On the other hand, the medium of the present embodiment allows better induction of neural stem cells to be achieved by not containing 2-ME.

In the first medium, the second medium, and the third medium, the amount of 2-ME may be 0 to 5 µmol/L, may be 0 to 1 µmol/L, or may be 0 to 0.1 µmol/L, or substantially no 2-ME maybe contained.

Decreasing the amount of 2-ME in the medium leads to better induction of neural stem cells.

It is preferable that the first supplement, the first medium, the second supplement, the second medium, the third supplement, and the third medium as mentioned above do not contain transferrin. Media in the related art may contain transferrin. On the other hand, the medium of the present embodiment allows better induction of neural stem cells to be achieved by not containing transferrin.

In the first medium, the second medium, and the third medium, the amount of transferrin may be 0 to 200 mg/L, may be 0 to 100 mg/L, or may be 0 to 10 mg/L, or substantially no transferrin may be contained.

Decreasing the amount of transferrin in the medium leads to better induction of neural stem cells.

It is preferable that the first supplement, the first medium, the second supplement, the second medium, the third supplement, and the third medium as mentioned above do not contain ethanolamine. Media in the related art may contain ethanolamine. On the other hand, the medium of the present embodiment allows better induction of neural stem cells to be achieved by not containing ethanolamine.

In the first medium, the second medium, and the third medium, the amount of ethanolamine may be 0 to 100 µmol/L. may be 0 to 50 µmol/L, or may be 0 to 20 µmol/L, or substantially no ethanolamine may be contained.

Decreasing the amount of ethanolamine in the medium leads to better induction of neural stem cells.

The first supplement may include, as active ingredients, insulin, selenic acid, basic fibroblast growth factor, and ascorbic acid or an ascorbic acid derivative.

The first supplement may include, as active ingredients, insulin, selenic acid, basic fibroblast growth factor, and ascorbic acid or an ascorbic acid derivative, and may further contain a solvent.

The second supplement may include, as active ingredients, insulin, selenic acid, basic fibroblast growth factor, a TGF-β receptor inhibitor, and a protein kinase C inhibitor.

The second supplement may include, as active ingredients, insulin, selenic acid, basic fibroblast growth factor, a TGF-β receptor inhibitor, and a protein kinase C inhibitor, and may further contain a solvent. The second supplement may further contain heparan sulfate as an active ingredient.

The third supplement may include, as active ingredients, insulin, selenic acid, basic fibroblast growth factor, a TGF-β receptor inhibitor, a protein kinase C inhibitor, and ascorbic acid or an ascorbic acid derivative.

The third supplement may include, as active ingredients, insulin, selenic acid, basic fibroblast growth factor, a TGF-β receptor inhibitor, a protein kinase C inhibitor, and ascorbic acid or an ascorbic acid derivative, and may further contain a solvent. The third supplement may further contain heparan sulfate as an active ingredient.

The active ingredient is an ingredient that is blended in the medium of the present embodiment and contributes to induction of neural stem cells or maintenance of neural stem cells.

As the solvent, water, a buffer solution such as phosphate buffered physiological saline, and tris-hydrochloric acid buffer solution, and an organic solvent such as dimethylsulfoxide are mentioned.

For absorbances of the first medium, the second medium, and the third medium, maximum values of the absorbances at a wavelength of 350 to 700 nm may be 0.7 or less, may be 0.6 or less, or may be 0.5 or less.

For the absorbances of the first medium, the second medium, and the third medium, maximum values of the absorbances at a wavelength of 500 to 700 nm may be 0.7 or less, may be 0.6 or less, or may be 0.5 or less.

A difference in the maximum values of the absorbances at the wavelength of 350 to 700 nm between the first medium and the second medium or the third medium may be 0.4 or less, may be 0.3 or less, or 0.2 or less.

An absorbance of the medium can be measured with a commercially available spectrophotometer by carrying out measurement for pure water as a control.

In media used for induction of neural stem cells in the related art, a very wide variety of ingredients are blended, so that these may increase absorbance values of the media and may hinder acquisition of an imaging image. In addition, there may be cases where absorbance values of a series of media used for inducing neural stem cells are not constant, and it becomes difficult to acquire an image under a stable condition.

On the other hand, the medium obtained by adding a supplement to a basal medium as mentioned above can be used for inducing neural stem cells while having a very simple composition as compared with medium ingredients used for inducing neural stem cells in the related art. Moreover, due to a simple composition, there is little possibility that a medium ingredient affects image acquisition, and it is possible to acquire an imaging image with high accuracy.

Furthermore, absorbance values of the first to third media used for inducing neural stem cells are substantially constant. Thus, it is possible to acquire an image under a stable condition. In addition, due to a simple composition, an influence of a test substance in a case of being used for screening or the like is easily detected, and assay of a test substance with high reproducibility is possible.

Pluripotent stem cells are cultured using the first medium, and then the cultured cells are cultured using the second medium, so that differentiation thereof into neural stem cells can be induced.

The respective ingredients contained in the first supplement can be used for pretreatment culture for inducing differentiation of pluripotent stem cells into neural stem cells while maintaining undifferentiation potency of the pluripotent stem cells to some extent. In addition, due to an action of ascorbic acid or an ascorbic acid derivative contained in the first supplement, cell damage caused by a seeding work and a subculture work can be decreased, and the cells can be induced to differentiate at a low mortality rate.

The respective ingredients contained in the second supplement can be used for a culture that induces differentiation of pluripotent stem cells into neural stem cells. The second supplement contains in addition to the respective ingredients of the first supplement as mentioned above, a TGF-β receptor inhibitor and a protein kinase C inhibitor. The TGF-β receptor inhibitor and the protein kinase C inhibitor are thought to act on differentiation into neural stem cells

It is preferable that the second supplement and the second medium do not contain ascorbic acid or an ascorbic acid derivative. The second supplement or the second medium allows differentiation induction efficiency for neural stem cells to be improved by not containing ascorbic acid or an ascorbic acid derivative.

The pluripotent stem cells cultured using the second medium may be further cultured using the third medium.

The third supplement may be provided as a supplement for a medium for subculture of neural stem cells. The third medium may be provided as a medium for subculture of neural stem cells.

Due to an action of ascorbic acid or an ascorbic acid derivative contained in the third supplement of the third medium, cell damage caused by a seeding work and a subculture work can be decreased, and neural stem cells can be maintained with a low mortality rate.

### <<Method for producing neural stem cells>>

The method for producing neural stem cells of the present embodiment includes a pluripotency-maintaining culture step, a first culture step, a second culture step, and a third culture step.

The method for producing neural stem cells of the present embodiment is carried out in the order of the pluripotency-maintaining culture step, the first culture step, the second culture step, and the third culture step.

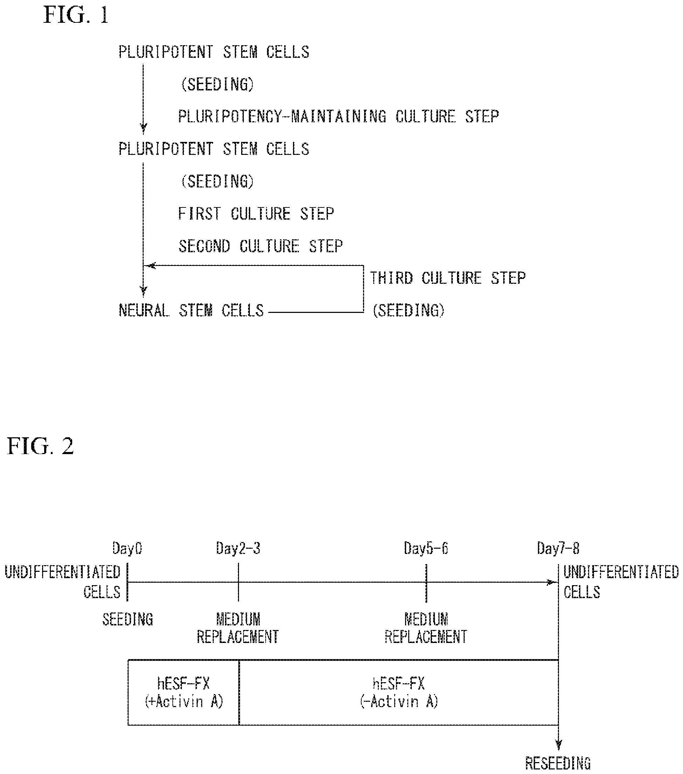

As shown in FIG. 1, pluripotent stem cells are induced to differentiate into neural stem cells through the first culture step and the second culture step, so that neural stem cells can be produced. The pluripotent stem cells and the neural stem cells can be brought into a substantially monolayer state.

In a case where the produced neural stem cells are subcultured, the neural stem cells can be maintained and cultured through the third culture step and the second culture step.

In the present specification, the "pluripotent stem cells" may be any undifferentiated cells that have undifferentiation potency capable of differentiating into at least neural stem cells. The pluripotent stem cells may be human pluripotent stem cells. As the pluripotent stem cells, germline stem cells (GS cells) isolated from testis immediately after birth (see, for example, Nature. 2008, 456: 344 to 349), mesenchymal stem cells, for example, bone marrow-derived stem cells such as iliac bone marrow and jawbone bone marrow, and adipose tissue-derived stem cells, and somatic cell-derived artificial pluripotent stem cells (or induced pluripotent stem cells (iPS cells)) having pluripotency similar to ES cells in which dedifferentiation of somatic cells of a subject itself has been induced by introducing a plurality of genes into the somatic cells such as skin cells can be mentioned. Specifically, iPS cells (Center for iPS Cell Research and Application, Kyoto University) obtained by introducing Oct3/4 gene, Klf4 gene, C-Myc gene, and Sox2 gene, iPS cells (Nat Biotechnol 2008; 26: 101 to 106) (Center for iPS Cell Research and Application, Kyoto University) obtained by introducing Oct3/4 gene, Klf4 gene, and Sox2 gene, 201B7 strain (HPS0063) (established by Center for iPS Cell Research and Application, Kyoto University, obtainable from RIKEN BioResource Center Cell Bank), 201B2 strain (established by Center for iPS Cell Research and Application, Kyoto University), 253G1 strain (HPS0002) (established by Center for iPS Cell Research and Application, Kyoto University, obtainable from RIKEN BioResource Center Cell Bank), iPS-TIG-120-4fl strain (JCRB134 1) (established by Center for iPS Cell Research and Application, Kyoto University, obtainable from National Institutes of Biomedical Innovation, Health and Nutrition JCRB Cell Bank), iPS-TIG114-4fl strain (JCRB 1437) (established by Center for iPS Cell Research and Application, Kyoto University, obtainable from National Institutes of Biomedical Innovation, Health and Nutrition JCRB Cell Bank), Tic strain (JCRB1331), Dotcom strain (JCRB1327), Squeaky strain (JCRB 1329), Toe strain (JCRB 1338), and Lollipop strain (JCRB1336) (established by National Center for Child Health and Development, obtainable from National Institutes of Biomedical Innovation, Health and Nutrition JCRB Cell Bank), UTA-1 strain (established by the University of Tokyo) and UTA-1-SF-2-2 strain (established by the University of Tokyo, obtainable from National Institutes of Biomedical Innovation, Health and Nutrition JCRB Cell Bank), and iPS DF19-9-7T strain (established by the University of Wisconsin, obtainable from WISC Bank) are exemplary examples.

"Culturing" in the present specification refers to growing cells in vitro. Culturing cells using a medium refers to culturing the cells in a slate where the medium and the cells are in contact with each other, such as culturing the cells in the medium or culturing the cells on the medium.

The medium may be in a gel state other than a liquid state, or may be in a liquid state.

Hereinafter, in the method for producing neural stem cells of the embodiment, the respective steps will be described.

### (Pluripotency-maintaining culture step)

The pluripotency-maintaining culture step is a step of culturing pluripotent stem cells using a pluripotency-maintaining medium. The pluripotency-maintaining medium is a medium in which pluripotent stem cells can be cultured in a state where undifferentiation potency capable of differentiating into at least neural stem cells is possessed. As such a medium, a medium known in the related art which is used for culturing pluripotent stem cells can be used.

As the pluripotency-maintaining medium, a medium reported in the related art or a commercially available medium can be used, and hESF9 medium (previously reported: doi: 10.1073/pnas.0806136105, Japanese Patent No. 5227318), hESF9a medium (previously reported: Stem Cell Res. 2010 Sep: 5(2): 157 to 169. doi: 10.1016/j.scr.2010.06.002), hESF9a2i medium (previously reported: PLoS One. 2013; 8(1): e54122. Published online 2013 Jan 21. doi:10.1371/journal.pone.0054122), hESF-FX medium (previously reported: PCT International Publication No. WO2012/104936 "METHOD FOR CULTURING HUMAN PLURIPOTENT STEM CELLS"), TeSR-E8 (STEMCELL Technologies), StemFit (registered trademark), hPSC Growth Medium DXF, and the like are mentioned.

FIG. 2 is a schematic diagram showing a procedure of the pluripotency-maintaining culture step. First, frozen-preserved undifferentiated cells are adjusted by a known method and then cultured by being seeded on hESF-FX medium containing activin A [hESF-FX (+ activin A) medium)] (pluripotency-maintaining medium). At 2 to 3 days after the start of the culture in the hESF-FX (+ activin A) medium, medium replacement was carried out from the hESF-FX medium to a hESF-FX (-activin A) medium (pluripotency-maintaining medium) to which activin A is not added, and the cells are cultured. At 5 to 6 days after the start of the culture, medium replacement is carried out, and the culture is continuously carried out in the hESF-FX (-activin A) medium.

According to the pluripotency-maintaining culture step, pluripotent stem cells can be cultured in a state where undifferentiation potency capable of differentiating into at least neural stem cells is possessed. In addition, medium ingredients for which adjustment has been made with supply sources for pluripotent stem cells are washed.

As a method for the above-mentioned adjustment, for example, 1) a method in which the pluripotent stem cells cultured in the above-mentioned pluripotency-maintaining culture step is liberated in EDTA which is dissolved in Dulbecco's Ca²⁺ and Mg²⁺ -free phosphate buffered physiological saline, 2) a method in which the cells are liberated by trypsin, gene-recombinant trypsin, trypsin/EDTA, collagenase, collagenase/trypsin, dispase, accumutase, or mechanical detachment, the liberated cells are recovered in the first medium to prepare a cell suspension, the cell suspension is precipitated at about 300 to 1,000 rpm and recovered, and then preparing the cell suspension again is repeated one time or a plurality of times, and 3) a method in which a culture is carried out on a gelatin-coated plate in the co-existence of a medium in which fetal bovine serum, DMEM, or DM/F12 is supplemented with KSR (KnockOut Serum Replacement, manufactured by G1BCO), L-glutamine, 2-mercaptoethanol, nonessential amino acid(s), and bFGF, and/or supporting cells (feeder cells) can be mentioned.

### (First culture step and second culture step)

The first culture step of the present embodiment is a step of culturing the pluripotent stem cells, which have been obtained in the pluripotency-maintaining culture step, using the first medium.

The second culture step of the present embodiment is a step of culturing the cells, which have been cultured in the first culture step, using the second medium, to obtain neural stem cells.

FIG. 3 is a schematic diagram showing a procedure of the first culture step and the second culture step. After being cultured in the pluripotency-maintaining culture step, the pluripotent stem cells are seeded on the first medium. Prior to seeding, for the pluripotent stem cells cultured in the pluripotency-maintaining culture step, an adjustment is preferably made by a known method. As a method for the adjustment, methods described in the pluripotency-maintaining culture step are mentioned. As the first medium, the first medium of the exemplary embodiment above is mentioned.

After seeding, medium replacement is carried out at 2 days after the start of the culture in the first medium, and the cells are cultured again in the second medium. The medium replacement herein is replacement of the first medium with the second medium As the second medium, the second medium of the exemplary embodiment above is mentioned. Subsequently, after 3 days from the start of the culture in the second medium, medium replacement is carried out, and the cells are continuously cultured in the second medium. In a case where the culture is continued for another 2 to 4 days after the medium replacement, neural stem cells are obtained from the pluripotent stem cells.

According to the first culture step and the second culture step, neural stem cells can be produced from undifferentiated cells.

### (Third culture step and second culture step)

The neural stem cells obtained in the second culture step can be further subcultured and maintained, or grown.

The third culture step of the present embodiment is a step of culturing the neural stem cells, which have been cultured in the second culture step, using the third medium. The third culture step of the present embodiment is also a step of subculturing the neural stem cells, which have been cultured in the second culture step, using the third medium.

FIG. 4 is a schematic diagram showing a procedure of the third culture step and the second culture step. After being cultured in the third culture step, the obtained neural stem cells are seeded on the third medium. Prior to seeding, for the neural stem cells cultured in the third culture step, an adjustment is preferably made by a known method. As a method of the adjustment, methods described in the pluripotency-maintaining culture step are mentioned. As the third medium, the third medium of the exemplary embodiment above is mentioned.

After seeding, medium replacement is carried out at 2 days after the start of the culture in the third medium, and the cells are cultured again in the second medium The medium replacement herein is replacement of the third medium with the second medium. Subsequently, after 3 days from the start of the culture in the second medium, medium replacement is carried out, and the cells are continuously cultured in the second medium.

According to the third culture step and the second culture step, neural stem cells can be subcultured and maintained, or grown.

As shown in FIG. 1, after undergoing the first culture step and the second culture step, the third culture step and the second culture step are carried out in a repeated manner, and the neural stem cells continue to be cultured.

After the second culture step, the neural stem cells may be preserved by a known method such as cryopreservation.

In the present embodiment, it is preferable that the cells to be cultured are cultured, in any of the above-mentioned steps, at a temperature of 33°C to 40°C, preferably 34°C to 39°C, and particularly preferably 37°C, in the presence of 1% to 20%, preferably 3% to 15%, and particularly preferably 8% to 10% carbon dioxide, under a high humidity of 75% or more, preferably 85% or more, and more preferably 95%, and particularly preferably 100%.

In the present embodiment, a monolayer culture may be carried out for the cells to be cultured. The monolayer culture is also referred to as a two-dimensional cell culture. As a method of carrying out the monolayer culture for cells, a method in which cells are cultured in a culture container whose inner wall is coated with a cell adhesion molecule is mentioned. As the cell adhesion molecule, fibronectin, polylysine, hyaluronic acid, laminin, collagen, vitronectin, elastin, and the like are mentioned, and one or two or more types of cell adhesion molecules may be used in combination.

The monolayer culture is preferably carried out in each of the above-mentioned steps, and the monolayer culture may be carried out under any one or more of the following conditions (A) to (C).
(A) In the first culture step, a monolayer culture is carried out for the pluripotent stem cells.
(B) In the second culture step, a monolayer culture is carried out for the cells cultured in the first culture step.
(C) In the third culture step, a monolayer culture is carried out for the neural stem cells obtained in the second culture step.

The method for producing neural stem cells according to the present embodiment may further include the following imaging step. In the method for producing neural stem cells according to the present embodiment, the first to third culture steps may further include the following imaging step.

Imaging step: An imaging step of acquiring images of cells which are being cultured in the first medium, second medium, or third medium

The method for producing neural stem cells according to the present embodiment may include, for example, any one or two or more of the following steps (i1) to (i3) as the imaging step.

Step (i 1) a first imaging step of acquiring images of the cells which are being cultured in the first medium, in the first culture step.

Step (i2) a second imaging step of acquiring images of the cells which are being cultured in the second medium, in the second culture step.

Step (i3) a third imaging step of acquiring images of cells which are being cultured in the third medium, in the third culture step.

The method for producing neural stem cells according to the present embodiment preferably includes any two or more of the steps (i1) to (i3). For example, the step (i1) and the step (i2), and/or the step (i3) may be included. In the first to third media according to the present embodiment, variation in absorbance is decreased. Thus, in a case of including any two or more of the steps (i1) to (i3), it is possible to stably acquire an image.

It is preferable to acquire an image in an optical manner. As an example, it is preferable to acquire an image including light having a wavelength of 350 to 700 nm, it is more preferable to acquire an image including light having a wavelength of 500 to 700 nm, and it is even more preferable to acquire an image including light having a wavelength of 600 to 700 nm.

As shown in Examples, the medium according to the present embodiment exhibits a lower absorbance over the entire region of a wavelength of 350 to 700 nm than media in the related art. Therefore, it is possible to stably acquire image data including light having a wide range of wavelengths. In addition, the medium according to the present embodiment exhibits remarkably decreased absorbance in a region of a wavelength of 350 to 500 nm and exhibits particularly decreased absorbance in a region of a wavelength of 350 to 400 nm, as compared with media in the related art. Therefore, as an example, it is preferable to acquire an image including light having a wavelength of 350 to 500 nm, and it is more preferable to acquire an image including light having a wavelength of 350 to 400 nm.

Due to the fact that the method for producing neural stem cells according to the present embodiment has the imaging step, it is possible to acquire information on a state of cells in the first to third culture steps, and it is possible to more accurately produce neural stem cells. As the information on a state of cells, cell morphology, various markers, and the like are mentioned.

In a case where images of cells which are being cultured using the first medium, the second medium, or the third medium are acquired, images of the medium are also acquired together with the cells. In these media according to the present embodiment, there is little possibility that a medium ingredient affects image acquisition, and observation can be made stably.

As a method for confirming whether or not the cells are cells maintaining undifferentiation potency and whether or not the cells are cells having properties as neural stem cells, a method of making a determination by detecting expression of an undifferentiation marker and/or by not detecting expression of a differentiation marker, using antibodies against various markers (proteins), a method of making a determination by detecting expression of a neurocyte marker and/or by not detecting expression of a neurocyte marker, a method of making a determination by detecting expression of various markers (genes), a method of observing morphological characteristics of cells, and a method of making a determination on whether or not the cells can exert an ability to differentiate into specific cells due to stimulation of a specific differentiation induction factor is an exemplary example.

In the method of making a determination by using markers, for example, in a case where an undifferentiated marker such as SSEA-3, SSEA-4, TRA-2-54, TRA-1-60, TRA-1-80, CD90, Nanog, Oct-3/4, and alkaline phosphatase is expressed, it can be determined that the cells maintain undifferentiation potency. Even in a case where a differentiation marker such as SSEA-1, CD105, CD56, and A2B5 is not expressed, it can be determined that the cells maintain undifferentiation potency.

In the method of making a determination by using markers, for example, in a case where a neural stem cell marker such as Nestin, Sox1, and Sox2 is expressed, it can be determined that the cells have been induced to neural stem cells. (At this time, in a case of also confirming that the undifferentiated marker is decreased or is not expressed, in addition to confirming expression of the neural stem cell marker, it may be determined that that the cells have been induced to neural stem cells.) In addition, in a case of also confirming that a neural marker such as Tuj1, MAP2, and AnkG is expressed slightly. and that a neural marker such as Tuj 1, MAP2, and AnkG is highly expressed in a subsequent culture, in addition to confirming expression of the neural stem cell marker, it may be determined that the cells have been induced to neural stem cells.

As described above, according to the method for producing neural stem cells of the embodiment, neural stem cells can be produced by culturing pluripotent stem cells using the first medium and the second medium of the embodiment.

The first medium contains ascorbic acid or an ascorbic acid derivative, so that due to an action of the ascorbic acid or ascorbic acid derivative, cell damage caused by a seeding work and a subculture work can be decreased, and it can induce differentiation of the cells at a low mortality rate.

In addition, by being cultured in the first medium and then cultured in the second medium, the cells can be induced to differentiate at a low mortality rate. It is recognized that the TGF-β receptor inhibitor and the protein kinase C inhibitor have an action to induce differentiation into neural stem cells. However, in a case where the pluripotent stem cells that have undergone the seeding work and the subculture work are directly cultured in the second medium without undergoing the first medium, an increased mortality rate of the cells is exhibited due to exposure to the TGF-p receptor inhibitor and the protein kinase C inhibitor. However, neural stem cells can be induced in a good state by being cultured in the first medium and then cultured in the second medium.

In addition, according to the method for producing neural stem cells of the embodiment, the neural stem cells can be subcultured using the third medium. The third medium contains ascorbic acid or an ascorbic acid derivative in addition to the ingredients of the second medium, so that due to an action of the ascorbic acid or ascorbic acid derivative, cell damage caused by a seeding work and a subculture work can be decreased, and the cells can be subcultured at a low mortality rate.

In addition, according to the respective media of the first medium, the second medium, and the third medium used in the present embodiment, light absorption is decreased, so that images of cells can be acquired accurately.

In addition, according to the respective media of the first medium, the second medium, and the third medium used in the present embodiment, in a state where a monolayer culture is carried out for the cells, neural stem cells in a substantially monolayer state can be produced.

In the related art, in inducing neural stem cells, a method of carrying out suspension culture of cells to form embryoid bodies and neurospheres so that neural stem cells are induced has been commonly used. However, according to this method, although neural stem cells can be produced, cells which are being cultured form an aggregate so that it is difficult to observe cells one by one.

On the other hand, according to the respective media used in the present embodiment, in a state where a monolayer culture is carried out for the cells, neural stem cells in a substantially monolayer state can be produced, so that images of individual cells can be accurately acquired. A state of the cells can be grasped, so that the neural stem cells can be produced efficiently.

### <C tilture method>

As a cell culture method in the embodiment of the present invention, there is provided a method for culturing cells in which the cells are cultured using the first medium. The cells may be pluripotent stem cells.

As the cell culture method in the embodiment of the present invention, there is provided a method for culturing cells in which the cells are cultured using the second medium.

As the cell culture method in the embodiment of the present invention, there is provided a method for culturing cells in which the cells are cultured using the third medium. The cells may be neural stem cells.

### <<Cell culture device>> not part of the present invention

FIG 5 is a schematic diagram showing an example of a configuration of a cell culture device of the present embodiment.

The cell culture device 1 of the present embodiment can be used in the method for producing neural stem cells of the embodiment as described above.

The cell culture device 1 includes an incubator 10 for culturing pluripotent stem cells, imaging means 20 for imaging cells C which are cultured in the incubator 10, medium replacement means 30 for replacing a first medium M1, a second medium M2, and a third medium M3 with each other in the incubator 10, and a controller 40.

An inside of the incubator 10 is maintained in an environment suitable for culturing cells. As the environment, the exemplary condition in the above-mentioned method for producing neural stem cells is mentioned.

In the incubator 10, a culture container 11 for culturing the cells is disposed, and the culture container 11 contains the cells C (pluripotent stem cells) and the first medium M1. The first culture step of the embodiment can be carried out by culturing the cells C which are pluripotent stem cells in the first medium M1.

After culturing the cells in the first culture step, the first medium M1 in the culture container 11 is replaced with the second medium M2 by the medium replacement means 30. The medium replacement means 30 has a medium suction nozzle 31 and a medium ejection nozzle 32. The first medium M1 is sucked and removed from an inside of the culture container 11 by the medium suction nozzle 31, and the second medium M2 is supplied into the culture container 11 by the medium ejection nozzle 32. By doing so, the culture container 11 is caused to contain the cells C, which have been cultured in the first medium M1, and the second medium M2. The second culture step of the embodiment can be carried out by culturing the cells C which have been cultured in the first medium M1, in the second medium M2.

The medium replacement means 30 further has a cell suction nozzle 33. The cells C that have undergone the second culture step in the culture container 11 are collected by the cell suction nozzle 33, and transferred to a culture container 12. The culture container 12 contains the third medium M3. The third culture step of the above embodiment can be carried out by culturing the cells C, which have undergone the second culture step, in the third medium M3.

After culturing the cells in the third culture step, the third medium in the culture container 12 may be replaced with the second medium M2 by the medium replacement means 30. The third medium is sucked and removed from an inside of the culture container 12 by the medium suction nozzle 31, and the second medium M2 is supplied into the culture container 12 by the medium ejection nozzle 32, so that the replacement is done. By doing so, the culture container 12 is caused to contain the cells C, which have been cultured in the third medium, and the second medium M2. Thus, by culturing this, the second culture step of the embodiment can be carried out

The cell culture device 1 further includes the controller 40. The controller 40 has a CPU 41 and a storage portion 42.

The cell culture device 1 includes the imaging means 20. The imaging means 20 includes a microscopic optical system 21 and an image sensor 22. The image data acquired by the image sensor 22 is sent to the controller 40, processed by the CPU 41, and a cell state is determined.

The controller 40 is connected to the medium replacement means 30, and the controller 40 controls a medium replacement operation to be made by the medium replacement means 30 depending on a determined cell state.

The image data acquired by the image sensor 22 is sent to the controller 40 and stored in the storage portion 42.

According to the culture device of the present specification, it is possible to carry out the method for producing neural stem cells of the embodiment. The cells cultured in the incubator are imaged by the imaging means, and a state of the cells is acquired and determined. Thus, it is possible to carry out medium replacement at an appropriate timing. At this time, according to the respective media of the first medium, the second medium, and the third medium used in the present embodiment, light absorption is decreased, so that images of cells can be acquired accurately and high-quality neural stem cells can be obtained efficiently.

### Examples

Next, the present invention will be described in more detail by way of Examples. However, the present invention is not limited to the following Examples.

Unless stated otherwise, a cell culture is carried out in an incubator at 37°C and 10% CO₂ concentration, in which the cell culture is carried out with a monolayer culture on a plate-coated plate as described below.

### (Plate coating)

Unless stated otherwise, in a cell culture, a plate obtained by coating a 6-well plate (model number MS-80060, manufactured by Sumitomo Bakelite Co., Ltd.) with Poly-L-Iysine (PLL) (SIGMA-ALDRICH. P4832, 55 ng/cm2 of POLY-L-LYSINE) and 2 µg/cm² of FN (SIGMA-ALDRICH, F1141-5MG, Fibronectin from bovine plasma > 1 mg/mL) was used.

### (Reagents)

Hereinafter, reagents used in the Examples are as follows
· mESF basal medium (manufacturer: Wako, model number: 136-17805)
· Insulin (Insulin Solution human, manufacturer: Sigma-Aldrich, model number: 19278)
· Transferrin (Apo-transferrin, manufacturer: Sigma-Aldrich, model number: T2252)
· Sodium selenite (manufacturer: Sigma-Aldrich, model number: 59133)
· Ethanolamine (2-Aminoethanol, manufacturer: Sigma-Aldrich, model number: E0135)
· Ascorbic acid (Ascorbic Acid stock solution, manufacturer: Wako, model number: 013-19641)
· FGF2 (rhFGF-2 (bFGF), manufacturer: KATAYAMA CHEMICAL, INC,)
· Heparan sulfate (Heparan sulfate sodium salt (manufacturer: Sigma-Aldrich, model number: H7640)
· SB 431542 (manufacturer: Tocris, model number: 1614)
· GF109203X (manufacturer: Tocris, model number: 0741)
· U0126 (manufacturer: Promega, model number: V1121)
· LY 294002 (manufacturer: Wako, model number: 129-04861)
· Activin A (Recombinant Human/Mouse/Rat Activin A, manufacturer: R *&* D, model number: 338-AC)
· 2-ME (2-Mercaptoethanol, manufacturer: Sigma-Aldrich, model number: M7522)
· rHSA (recombinant human albumin, manufacturer: Sigma-Aldrich, model number: A7736)
· Oleic acid (oleic acid, manufacturer: Sigma-Aldrich, model number: O 1383)
· BSA (manufacturer: Sigma-Aldrich, model number: A 8806)

### <1. Examination for medium>

### (Examination (1) for supplement ingredients)

In order to develop a condition for induction of differentiation which includes only essential supplements at a minimum, for combinations of 7 ingredients to be added, under a serum-free culture condition, to mESF medium which is a basal medium, 34 conditions shown in Table 1 were examined.

**[Table 1]**

| Condition | 1 | 2 | 3 | | 4 | 5 | 6 | 7 | | 8 | | 9 | 10 | 11 | 12 | | 13 | | 14 | 15 | 16 | 17 | | 18 | 19 |
|---|---|---|---|---|---|---|---|---|---|---|---|---|---|---|---|---|---|---|---|---|---|---|---|---|---|
| Insulin of 10 mg/L | ○ | ○ | ○ | | ○ | ○ | ○ | | | | | | | | | | | | ○ | ○ | ○ | ○ | | ○ | ○ |
| Transferrin of 5 mg/L | | | | | | | | ○ | | ○ | | ○ | ○ | ○ | ○ | | ○ | | ○ | ○ | ○ | ○ | | ○ | ○ |
| Sodium selenite of 20 µmol/L | | ○ | | | | | | | | ○ | | | | | | | | | | | | | | | |
| Ethunolamine of 10 µmol/L | | | | | | | | | | | | ○ | | | | | | | | ○ | | | | | |
| 2·ME of 10µmol/L | | | ○ | | | | | | | | | ○ | ○ | | | | | | | | ○ | | | | |
| USA of 1g/L | | | | | ○ | | | | | | | | | ○ | | | | | | | | ○ | | | |
| Oleic acid of 9.4 mg/L (used as a solution conjugated with rHSA of 1 g/L) | | | | | | ○ | | | | | | | | | ○ | | | | | | | | | ○ | |
| Heparan sulfate of 100 µmol/L | | | | | | | ○ | | | | | | | | | | ○ | | | | | | | | ○ |

| Condition | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|
| Insulin of 10 mg/L | ○ | ○ | ○ | ○ | ○ | ○ | ○ | ○ | ○ | ○ | ○ | ○ | ○ | ○ | ○ |
| Transferrin of 5 mg/L | ○ | ○ | ○ | ○ | ○ | ○ | ○ | ○ | ○ | ○ | ○ | ○ | ○ | ○ | ○ |
| Sodium selenite of 20 µmol/L | ○ | ○ | ○ | ○ | ○ | ○ | ○ | ○ | ○ | ○ | ○ | ○ | ○ | ○ | ○ |
| Ethanolamine of 10 µmol/L | | ○ | | | | | | | | ○ | ○ | ○ | ○ | ○ | ○ |
| 2·ME of 10µmol/L | | | ○ | | | | ○ | | | | ○ | | | ○ | ○ |
| rHSA of 1g/L | | | | ○ | | | | ○ | | | | ○ | | ○ | |
| Oleic acid of 94 mg/L(used as a solution conjugated wirh rHSA of 2g/L) | | | | | ○ | | | | ○ | | | | ○ | | ○ |
| Heparan sulfate of 100 µmol/L | | | | | | ○ | | | ○ | ○ | ○ | ○ | ○ | ○ | ○ |

| | | | | | | | | | | | | | | | |
|---|---|---|---|---|---|---|---|---|---|---|---|---|---|---|---|
| *In Table 1, "○" represents that the marked ingredient is contained in a medium at a final concentration indicated in Table 1. | | | | | | | | | | | | | | | |

### Reference Example

Using 34 types of media shown in Table 1, human ES cells 119 were cultured in the respective media in such a manner that a uniform seeding concentration is ensured, and DAPI staining was carried out to count the number of cells.

The results are shown in FIG. 6. As is clearly shown from the graph of FIG 6, it was clearly shown that a combination of insulin and sodium selenite brings about very good results for culturing human ES cells H9.

### (Examination (2) of supplement ingredients)

Next, to the medium (mESFis) which is obtained in the above examination results and includes mESF, insulin, and sodium selenite, various inhibitors were added under four conditions shown in Table 2, the human ES cells H9 were cultured, immunostaining was carried out using a Tuj1 antibody which is a marker of neural cells, and an effect of inducing differentiation into neural cells was examined. Various inhibitors were added to the mESFis so that the respective final concentrations indicated in Table 2 are achieved.

**[Table 2]**

| Condition | 1 | 2 | 3 | 4 |
|---|---|---|---|---|
| GF109203X | 2.5 µmol/L | 2.5 µmol/L | 2.5 µmol/L | 2.5 µmol/L |
| U0126 | | 5 µmol/L | | |
| LY294002 | | | 20 µmol/L | |
| SB431542 | | | | 10 µmol/L |

The results are shown in FIG. 7. Even in DMSO used as a solvent for various inhibitors, Tuj1 positive cells may be increased. However, a combination of GF109203X and SB431542 exhibited increased Tuj1 positive cell counts and a high effect of inducing differentiation into neural cells.

### (Examination (3) of supplement ingredients)

Next, examination was made for an influence of other ingredients that can be added to the mESFis.

Various ingredients were added to the mESFis under four conditions shown in Table 3, human ES cells H9 were cultured, immunostaining was carried out using the Tuj1 antibody which is a marker of neural cells, and an effect of inducing differentiation into neural cells was examined. In addition, lengths of nerve fibers of the cells were measured. Various ingredients were added to the mESFis so that the respective final concentrations indicated in Table 3 are achieved.

**[Table 3]**

| Condition | 1 (No addition) | 2 | 3 | 4 |
|---|---|---|---|---|
| FGF-2 | | 100 ng/mL | | 100 ng/mL |
| SB431542 | | 10 µmol/L | | 10 µmol/L |
| GF109203X | | 2.5 µmol/L | | 2.5 µmol/L |
| Transferrin | | | 5 mg/L | 5 mg/L |
| Ethanolamine | | | 10 µmol/L | 10 µmol/L |
| BSA | | | 1 g/L | 1 g/L |

The results are shown in FIG. 8. In a case where transferrin and ethanolamine are present in the mESFis, it was found that elongation of nerve fibers is inhibited. An influence of BSA was not particularly observed. It was proven that a combination of SB431542 and GF109203X promotes Tuj 1 positive cell counts and extension of nerve fibers.

### (Examination (4) of supplement ingredients)

Next, using FGF-2 and heparan sulfate sodium salt in combination was examined.

Human ES cells H9 were cultured for 1 day in medium 1 as described below. Thereafter, the medium 1 was replaced with each of the media having conditions shown in Table 4, and the culture was continued. For FGF-2 of 0 to 100 ng/mL, a medium was prepared such that FGF-2 is contained at a concentration of 0 ng/mL, I ng/mL, 2 ng/mL, 5 ng/mL, 10 ng/mL, or 100 ng/mL. Thereafter, a phase difference living cell image was acquired, and a determination was made for cell viability and cell morphology.

**[Table 4]**

| Condition | | 1 | 2 |
|---|---|---|---|
| Basal medium | | mESF | mESF |
| Supplement | Insulin | 10 µg/mL | 10 µg/mL |
| | Sodium selenite | 20 nmol/L | 20 nmol/L |
| | FGF-2 | 0 to 100 ng/mL | 0 to 100 ng/mL |
| | Heparan sulfate | | 100 ng/mL |
| | SB431542 | 10 µmol/L | 10 µmol/L |
| | GFJ09203X | 2.5 µmol/L | 2.5 µmol/L |

The results are shown in FIGS. 9 and 10. A value on an upper left of the drawing represents a concentration (ng/mL) of FGF-2 contained in the medium, FIG. 9 shows the results with a condition in which the medium does not contain heparan sulfate sodium salt (1 in Table 4), and FIG. 10 shows the results with a condition in which the medium contains heparan sulfate sodium salt (2 in Table 4). From a comparison of FIG. 9 and FIG. 10, it was shown that differentiation into neural stem cells can be induced stably with a low concentration of FGF-2 by using FGF-2 and heparan sulfate sodium salt in combination

### <2. Culture of neural stem cells>

### (Preparation of pluripotency-maintaining medium)

As a pluripotency-maintaining medium, the respect ingredients were added to mESF so that concentrations thereof in the medium become final concentrations shown in Table 5, and hESF-FX was prepared.

**[Table 5]**

| | | hESF-FX |
|---|---|---|
| Basal medium | | mESF |
| Supplement | Ascorbic acid | 0.1 mg/L |
| | Insulin | 10 µg/mL |
| | Sodium selenite | 20 nmol/L |
| | 2-ME | 10 nmol/L |
| | Ethanolamine | 10 nmol/L |
| | Apo-transferrin | 5 µg/mL |
| | Oleic acid (used as a solution conjugated with rHSA of 1 mg/mL) | 9.4 µg/mL |
| | FGF-2 | 5 ng/mL |
| | Activin A | 2 ng/mL |

### (Preparation of first medium, second medium, third medium)

Taking the results of the above-described <1. Examination for medium > into consideration, media used for producing neural stem cells were prepared.

Supplement ingredients were added to the mESF so that concentrations thereof in the mESF basal medium become final concentrations in Table 6. Medium 1 (first medium), medium 2 (second medium), and medium 3 (third medium) in which the respective ingredients were blended in the mESF medium with compositions shown in Table 6 were obtained.

**[Table 6]**

| | | Medium 1 | Medium 2 | Medium 3 |
|---|---|---|---|---|
| | | First medium | Second medium | Third medium |
| Basal medium | | mESF | mESF | mESF |
| Supplement | Insulin | 10 µg/mL | 10 µg/mL | 10 µg/mL |
| | Sodium selenite | 20 nmol/L | 20 nmol/L | 20 mnol/L |
| | Ascorbic acid | 0.1 mg/mL | | 0.1 mg/mL |
| | FGF-2 | 10 ng/mL | 10 ng/mL | 10 ng/mL |
| | Heparan sulfate | | 100 ng/mL | 100 ng/mL |
| | SB431542 | | 10 µmol/L | 10 µmol/L |
| | GF109203X | | 2.5 µmol/L | 2.5 µmol/L |
| Amount of albumin in medium | | 0 mg/L | 0 mg/L | 0 mg/L |

### (Neural stem cell induction)

Subsequently, human iPS cell Tic strain (JCRB1331) was suspended in hESF-FX medium, seeded on the coated 6-well plate (seeding density of 0.5 to 5 × 10⁴/well), and cultured in hESF-FX medium for 7 to 8 days while carrying out medium replacement in the hESF-FX medium at a frequency of once every 2 to 3 days.

The cells cultured in the hESF-FX medium were detached with Accutase (Millipore, Cat#SF006) and recovered from the flask. The cells were seeded at a seeding density of 0.5 to 5 × 10⁴/well on a 6-well plate coated with FN and PLL in the same manner as described above, and cultured for 2 days in medium 1. Then, the medium was replaced with medium 2, and a culture was carried out in the medium 2 for 7 to 9 days while carrying out medium replacement in the hESF-FX medium at a frequency of once every 2 to 3 days.

For the cells which were being cultured in the medium 2, expression of proteins of various markers shown in FIG. 11 was observed with a fluorescence microscope. In FIG. 11, Day 2 corresponds to the day when the culture started in the medium 2.

As the culture in the medium 2 progressed, expression of Oct3/4 and Otx2 which are undifferentiated markers disappeared. In addition, expression of Nestin which is a neural stem cell marker, and of Tujl, MAP2, and AnkG which are neural markers was observed. PAX 6 was used as a forward marker, and GFAP was used as a negative control.

From the results shown in FIG. 11, it was shown that neural stem cells could be induced by the culture.

In addition, images of the cells which are being cultured using the media according to the embodiment of the present invention could be acquired well.

The cells cultured in the medium 2 were detached with Accutase and recovered from a microplate. The cells were suspended in the medium 3, seeded at a seeding density of 0.5 to 5 × 10⁴/well on a 6-well plate coated with FN and PLL in the same manner as described above, and cultured for 2 days in the medium 3. Then, the medium was replaced with medium 2, and a culture was carried out in the medium 2 for 8 days while carrying out medium replacement at a frequency of once every 2 to 3 days.

It was shown that subculture of neural stem cells is possible by seeding the cells on the medium 3 and then culturing the cells in the medium 2.

### <3. Absorbance of medium>

As shown in the protocol (UKN 1) of Non-Patent Document 3, in a series of steps from undifferentiation maintenance to induction of differentiation into neural cells, media obtained by changing compositional ratios of "a medium to which KSR was added to DMEM-F12" and "a medium supplemented with N2" depending on each of the steps are commonly used. The results obtained by measuring absorbances of supernatants of the media with a spectrophotometer (manufacturer: JASCO Corporation, model: V-630, measurement mode: Abs) in these series of differentiation induction processes are shown in FIG. 12.

In the graph of FIG. 12, it was shown that there are large variations in absorbance of the media in the respective steps. This is considered to result from involvement of an influence caused by difference in medium ingredients as well as an influence caused by difference in various ingredients such as metabolites generated from cells during culturing. In making an observation under a microscope, in a case of being observed under a constant observation condition, unstable brightness is exhibited due to such variations in absorbance. Thus, a medium with less variation in absorbance is desirable.

For the mESF medium (the above-mentioned medium 2) according to the present embodiment to which a supplement for inducing a cell to differentiate into neural stem cells is added, the results obtained by measuring absorbance of a supernatant of the medium in a differentiation induction process are shown in FIG. 13.

The medium according to the present embodiment was proven to be a medium which exhibits less variation in absorbance as compared with the medium shown in FIG. 12 and is more suitable for observation.

The present invention is not limited by the respective embodiments, and is limited only by the scope of claims.

### Reference Signs List

1: cell culture device
10: incubator
11, 12: culture container
20: imaging means
21: microscopic optical system
22: image sensor
30: medium replacement means
31: medium suction nozzle
32: medium ejection nozzle
33: cell suction nozzle
40: controller
42: storage portion
M1: first medium
M2: second medium
M3: third medium
C: cells

## Claims

1. Use of a medium supplement for the induction of differentiation from a cell into neural stem cell, comprising:
a TGF-β receptor inhibitor; and
a protein kinase C inhibitor,
wherein the medium supplement does not contain ascorbic acid or an ascorbic acid derivative and/or does not contain albumin, 2-mercaptoethanol, transferrin, or ethanolamine.

2. The use according to Claim 1, wherein the medium supplement further comprises at least one selected from the group consisting of insulin, selenic acid, basic fibroblast growth factor, and heparan sulfate.

3. The use according to Claim 1 or 2, wherein the medium supplement is added to a basal medium not containing ascorbic acid and the ascorbic acid derivative.

4. Use of a medium supplement set for the induction of differentiation from a cell into neural stem cell, comprising:
a first supplement containing ascorbic acid or an ascorbic acid derivative;
a second supplement containing a TGF-β receptor inhibitor and a protein kinase C inhibitor, and not containing ascorbic acid and the ascorbic acid derivative;
a third supplement containing ascorbic acid or the ascorbic acid derivative, the TGF-β receptor inhibitor, and the protein kinase C inhibitor.

5. The use according to Claim 4,
wherein the first supplement further contains at least one selected from the group consisting of insulin, selenic acid, and basic fibroblast growth factor;
wherein the second supplement further contains at least one selected from the group consisting of insulin, selenic acid, basic fibroblast growth factor, and heparan sulfate; and/or
wherein the third supplement further contains at least one selected from the group consisting of insulin, selenic acid, basic fibroblast growth factor, and heparan sulfate.

6. Use of a medium for the induction of differentiation from a cell into neural stem cell, comprising:
a TGF-β receptor inhibitor; and
a protein kinase C inhibitor,
which does not contain ascorbic acid or an ascorbic acid derivative and/or does not contain albumin, 2-mercaptoethanol, transferrin, or ethanolamine.

7. The use according to Claim 6, wherein the medium further comprises at least one selected from the group consisting of insulin, selenic acid, basic fibroblast growth factor, and heparan sulfate.

8. A method for producing neural stem cells, comprising:
culturing cells using a medium that contains a TGF-β receptor inhibitor and a protein kinase C inhibitor.

9. The method for producing neural stem cells according to Claim 8,
wherein the medium further contains at least one selected from the group consisting of insulin, selenic acid, basic fibroblast growth factor, and heparan sulfate, and does not contain ascorbic acid or an ascorbic acid derivative.

10. The method for producing neural stem cells according to Claim 8 or 9, further comprising:
culturing the cells using a medium that contains ascorbic acid or the ascorbic acid derivative, before culturing the cells using the medium that contains the TGF-β receptor inhibitor and the protein kinase C inhibitor.

11. The method for producing neural stem cells according to any one of Claims 8 to 10,
wherein the medium containing ascorbic acid or the ascorbic acid derivative, further contains at least one selected from the group consisting of insulin, selenic acid, and basic fibroblast growth factor.

12. The method for producing neural stem cells according to any one of Claims 8 to 11, further comprising:
culturing the cells using a medium that contains ascorbic acid or the ascorbic acid derivative, the TGF-β receptor inhibitor, and the protein kinase C inhibitor, after culturing the cells using the medium that contains the TGF-β receptor inhibitor and the protein kinase C inhibitor.

13. The method for producing neural stem cells according to any one of Claims 8 to 12,
wherein the cells are pluripotent stem cells.

14. Use for the induction of differentiation from a cell into neural stem cell of a medium kit, comprising:
a basal medium; and
the medium supplement according to any one of Claims 1 to 3 or the medium supplement set according to Claim 4 or 5.

## Patentansprüche

1. Verwendung eines Mediumzusatzes für die Induktion der Differenzierung einer Zelle in eine neurale Stammzelle, umfassend:
einen TGF-β-Rezeptor-Inhibitor; und
einen Proteinkinase-C-Inhibitor,
wobei der Mediumzusatz keine Ascorbinsäure oder ein Ascorbinsäurederivat enthält und/oder kein Albumin, 2-Mercaptoethanol, Transferrin, oder Ethanolamin enthält.

2. Verwendung gemäß Anspruch 1, wobei der Mediumzusatz ferner mindestens eines ausgewählt aus der Gruppe bestehend aus Insulin, Selensäure, basischem Fibroblasten-Wachstumsfaktor und Heparansulfat umfasst.

3. Verwendung gemäß Anspruch 1 oder 2, wobei der Mediumzusatz zu einem Basalmedium hinzugegeben wird, das keine Ascorbinsäure und kein Ascorbinsäurederivat enthält.

4. Verwendung eines Mediumzusatz-Sets für die Induktion der Differenzierung einer Zelle in eine neurale Stammzelle, umfassend:
einen ersten Zusatz, der Ascorbinsäure oder ein Ascorbinsäurederivat enthält;
einen zweiten Zusatz, der einen TGF-β-Rezeptor-Inhibitor und einen Proteinkinase-C-Inhibitor enthält, und die Ascorbinsäure und das Ascorbinsäurederivat nicht enthält;
einen dritten Zusatz, der die Ascorbinsäure oder das Ascorbinsäurederivat, den TGF-β-Rezeptor-Inhibitor
und den Proteinkinase-C-Inhibitor enthält.

5. Verwendung gemäß Anspruch 4,
wobei der erste Zusatz ferner mindestens eines ausgewählt aus der Gruppe bestehend aus Insulin, Selensäure und basischem Fibroblasten-Wachstumsfaktor enthält;
wobei der zweite Zusatz ferner mindestens eines ausgewählt aus der Gruppe bestehend aus Insulin, Selensäure, basischem Fibroblasten-Wachstumsfaktor und Heparansulfat enthält; und/oder
wobei der dritte Zusatz ferner mindestens eines ausgewählt aus der Gruppe bestehend aus Insulin, Selensäure, basischem Fibroblasten-Wachstumsfaktor und Heparansulfat enthält.

6. Verwendung eines Mediums für die Induktion der Differenzierung einer Zelle in eine neurale Stammzelle, umfassend:
einen TGF-β-Rezeptor-Inhibitor; und
einen Proteinkinase-C-Inhibitor,
welches keine Ascorbinsäure oder ein Ascorbinsäurederivat enthält und/oder kein Albumin, 2-Mercaptoethanol, Transferrin, oder Ethanolamin enthält.

7. Verwendung gemäß Anspruch 6, wobei das Medium ferner mindestens eines ausgewählt aus der Gruppe bestehend aus Insulin, Selensäure, basischem Fibroblasten-Wachstumsfaktor und Heparansulfat umfasst.

8. Verfahren zur Herstellung neuraler Stammzellen, umfassend:
Kultivierung von Zellen unter Verwendung eines Mediums, das einen TGF-β-Rezeptor-Inhibitor und einen Proteinkinase-C-Inhibitor enthält.

9. Verfahren zur Herstellung neuraler Stammzellen gemäß Anspruch 8, wobei das Medium ferner mindestens eines ausgewählt aus der Gruppe bestehend aus Insulin, Selensäure, basischem Fibroblasten-Wachstumsfaktor und Heparansulfat enthält und keine Ascorbinsäure oder ein Ascorbinsäurederivat enthält.

10. Verfahren zur Herstellung neuraler Stammzellen gemäß Anspruch 8 oder 9, ferner umfassend:
Kultivierung der Zellen unter Verwendung eines Mediums, das Ascorbinsäure oder das Ascorbinsäurederivat enthält, vor der Kultivierung der Zellen unter Verwendung des Mediums, das den TGF-β-Rezeptor-Inhibitor und den Proteinkinase-C-Inhibitor enthält.

11. Verfahren zur Herstellung neuraler Stammzellen gemäß irgendeinem der Ansprüche 8 bis 10, wobei das Medium, das Ascorbinsäure oder das Ascorbinsäurederivat enthält, ferner mindestens eines ausgewählt aus der Gruppe bestehend aus Insulin, Selensäure, und basischem Fibroblasten-Wachstumsfaktor enthält.

12. Verfahren zur Herstellung neuraler Stammzellen gemäß irgendeinem der Ansprüche 8 bis 11, ferner umfassend:
Kultivierung der Zellen unter Verwendung eines Mediums, das Ascorbinsäure oder das Ascorbinsäurederivat, den TGF-β-Rezeptor-Inhibitor und den Proteinkinase-C-Inhibitor enthält, nach Kultivierung der Zellen unter Verwendung des Mediums, das den TGF β-Rezeptor-Inhibitor und den Proteinkinase-C-Inhibitor enthält.

13. Verfahren zur Herstellung neuraler Stammzellen gemäß irgendeinem der Ansprüche 8 bis 12, wobei die Zellen pluripotente Stammzellen sind.

14. Verwendung eines Medium-Kits für die Induktion der Differenzierung einer Zelle in eine neurale Stammzelle, umfassend:
ein Basalmedium; und
den Mediumzusatz gemäß irgendeinem der Ansprüche 1 bis 3 oder das Mediumzusatz-Set gemäß Anspruch 4 oder 5.

## Revendications

1. Utilisation d'un complément de milieu pour l'induction de différenciation d'une cellule en une cellule souche neuronale, comprenant :
un inhibiteur du récepteur TGF-β ; et
un inhibiteur de la protéine kinase C,
dans laquelle le complément de milieu ne contient pas d'acide ascorbique ou un dérivé de l'acide ascorbique et/ou ne contient pas d'albumine, de 2-mercaptoéthanol, de transferrine, ou d'éthanolamine.

2. Utilisation selon la revendication 1, dans laquelle le complément de milieu comprend en outre au moins un élément sélectionné dans le groupe constitué de l'insuline, de l'acide sélénique, du facteur de croissance de base des fibroblastes et du sulfate d'héparane.

3. Utilisation selon la revendication 1 ou la revendication 2, dans laquelle le complément de milieu est ajouté à un milieu basal ne contenant pas d'acide ascorbique et de dérivé de l'acide ascorbique.

4. Utilisation d'un jeu de compléments de milieu pour l'induction de différenciation d'une cellule en une cellule souche neuronale, comprenant :
un premier complément contenant de l'acide ascorbique ou un dérivé de l'acide ascorbique ;
un deuxième complément contenant un inhibiteur du récepteur TGF-β et un inhibiteur de la protéine kinase C et ne contenant pas d'acide ascorbique et de dérivé de l'acide ascorbique ;
un troisième complément contenant de l'acide ascorbique ou le dérivé de l'acide ascorbique, l'inhibiteur du récepteur TGF-β et l'inhibiteur de la protéine kinase C.

5. Utilisation selon la revendication 4,
dans laquelle le premier complément contient en outre au moins l'un sélectionné dans le groupe constitué de l'insuline, de l'acide sélénique et du facteur de croissance de base des fibroblastes ;
dans laquelle le deuxième complément contient en outre au moins l'un sélectionné dans le groupe constitué de l'insuline, de l'acide sélénique, du facteur de croissance de base des fibroblastes et du sulfate d'héparane ; et/ou
dans laquelle le troisième complément contient en outre au moins l'un sélectionné dans le groupe constitué de l'insuline, de l'acide sélénique, du facteur de croissance de base des fibroblastes et du sulfate d'héparane.

6. Utilisation d'un milieu pour l'induction de différenciation d'une cellule en une cellule souche neuronale, comprenant :
un inhibiteur du récepteur TGF-β ; et
un inhibiteur de la protéine kinase C,
qui ne contient pas d'acide ascorbique ou un dérivé de l'acide ascorbique et/ou ne contient pas d'albumine, de 2-mercaptoéthanol, de transferrine, ou d'éthanolamine.

7. Utilisation selon la revendication 6, dans laquelle le milieu comprend en outre au moins l'un sélectionné dans le groupe constitué de l'insuline, de l'acide sélénique, du facteur de croissance de base des fibroblastes et du sulfate d'héparane.

8. Procédé de production de cellules souches neuronales, comprenant :
la culture de cellules à l'aide d'un milieu qui contient un inhibiteur du récepteur TGF-β et un inhibiteur de la protéine kinase C.

9. Procédé de production de cellules souches neuronales selon la revendication 8, dans lequel le milieu contient en outre au moins l'un sélectionné dans le groupe constitué de l'insuline, de l'acide sélénique, du facteur de croissance de base des fibroblastes et du sulfate d'héparane, et ne contient pas d'acide ascorbique ou un dérivé de l'acide ascorbique.

10. Procédé de production de cellules souches neuronales selon la revendication 8 ou la revendication 9, comprenant en outre :
la culture des cellules à l'aide d'un milieu qui contient de l'acide ascorbique ou le dérivé de l'acide ascorbique, avant de mettre en culture les cellules à l'aide du milieu qui contient l'inhibiteur du récepteur TGF-β et l'inhibiteur de la protéine kinase C.

11. Procédé de production de cellules souches neuronales selon l'une quelconque des revendications 8 à 10, dans lequel le milieu contenant l'acide ascorbique ou le dérivé d'acide ascorbique, contient en outre au moins l'un sélectionné dans le groupe constitué de l'insuline, de l'acide sélénique et du facteur de croissance de base des fibroblastes.

12. Procédé de production de cellules souches neuronales selon l'une quelconque des revendications 8 à 11, comprenant en outre :
la culture des cellules à l'aide d'un milieu qui contient de l'acide ascorbique ou le dérivé de l'acide ascorbique, l'inhibiteur du récepteur TGF-β et l'inhibiteur de la protéine kinase C, après la culture des cellules à l'aide d'un milieu qui contient l'inhibiteur du récepteur TGF-β et l'inhibiteur de la protéine kinase C.

13. Procédé de production de cellules souches neuronales selon l'une quelconque des revendications 8 à 12, dans lequel les cellules sont des cellules souches pluripotentes.

14. Utilisation pour l'induction de différenciation d'une cellule en une cellule souche neuronale d'un kit de milieu, comprenant :
un milieu basal ; et
le complément de milieu selon l'une quelconque des revendications 1 à 3 ou le jeu de compléments de milieu selon la revendication 4 ou la revendication 5.
